(19) Europäisches Patentamt
European Patent Office
Office européen des brevets

(11) EP 1 295 879 A1

(12) # EUROPEAN PATENT APPLICATION
published in accordance with Art. 158(3) EPC

(43) Date of publication:
**26.03.2003 Bulletin 2003/13**

(21) Application number: **01936940.4**

(22) Date of filing: **11.06.2001**

(51) Int Cl.⁷: **C07D 403/06**, C07D 405/06, C07D 409/06, C07D 413/06

(86) International application number:
**PCT/JP01/04887**

(87) International publication number:
**WO 01/096329 (20.12.2001 Gazette 2001/51)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.06.2000 JP 2000176844**

(71) Applicant: **SHIONOGI & CO., LTD.**
**Osaka-shi, Osaka 541-0045 (JP)**

(72) Inventor: **SATO, Akihiko, c/o Shionogi & Co., Ltd.**
**Settsu-shi, Osaka 566-0022 (JP)**

(74) Representative: **VOSSIUS & PARTNER**
**Siebertstrasse 4**
**81675 München (DE)**

(54) **MEDICINAL COMPOSITIONS CONTAINING PROPENONE DERIVATIVES**

(57) A combination of an integrase inhibitor with an anti-retrovirus active substance and medical compositions containing the same as the active ingredients are found.

EP 1 295 879 A1

## Description

### Technical Field

The present invention relates to medicinal compositions containing two or more anti-retrovirus active substances, in detail medicinal compositions containing propenone derivatives.

### Background Art

AIDS (acquired immunodeficiency syndrome) is an intractable disease cased by human immunodeficiency virus (HIV), and the development of a useful treating agent and therapy therefore are expected. As a treating agent for AIDS, a reverse transcriptase inhibitor and a protease inhibitor, have already been authorized and in clinical field, a combination therapy rather than a single administration of them is used as a standard method because of the appearance of resistant virus, reduction of side-effect, etc.

In combination therapy, the use of medicines having different action mechanism is more recommended than that of the same type of medicines. The combination of such different type of medicines can strongly inhibit the replication of retrovirus though their synergism. For example, preferable is a combination of nucleoside type reverse transcriptase inhibitor and non-nucleoside type reverse transcriptase inhibitor, a combination of reverse transcriptase inhibitor (nucleoside type reverse transcriptase inhibitor or non-nucleoside type reverse transcriptase inhibitor) and protease inhibitor, and the like.

As examples of the synergistic effect exhibited by a combination of plural anti-human immunodeficiency virus active substances, a combination of lamivudine (3TC) as nucleoside type reverse transcriptase inhibitor and {[(benzoxazol-2-yl)methyl]amino}-5-alkyl-6-alkyl-2-(1H)-pyridinone as non-nucleoside type reverse transcriptase inhibitor is disclosed in JP7-508997, and a combination of lamivudine (3TC) as nucleoside type reverse transcriptase inhibitor and 11-cyclopropyl-5,11-dihydro-4-methyl-6H-dipyrido[3,3-b;21,31-e][1,4]diazepin-6-one as non-nucleoside type reverse transcriptase inhibitor is disclosed in JP national publication (TOKUHYO) 7-508998. Other examples of the combination of anti-human immunodeficiency virus active substances are disclosed in JP national publication (TOKUHYO) 2000-503986, WO98/20888, JP national publication (TOKUHYO) 11-507632, JP national publication (TOKUHYO) 11-508884, JP national publication (TOKUHYO) 9-507859, and the like.

Furthermore, Combivir is sold at the present as the medicinal mixture for one of the combination therapy, which is a mixture of zidovudine (AZT) and lamivudine (3TC). Therefore, it has been desired to develop a combination of a medicine (for example, integrase inhibitor), which has a different action mechanism from reverse transcriptase inhibitor and protease inhibitor, with an anti-retrovirus active substance, as well as a medicinal composition containing their medicines as active ingredient s.

### Disclosure of Invention

The present invention has found that a synergistic effect is exhibited by a combined administration of a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances, wherein these medicines enhance their anti-retrovirus activities.

As an embodiment, the present invention provides an anti-retrovirus composition which contains as an active ingredient a compound represented by the formula (I): A-C(=O)-CH=C(OH)-B wherein A and B are as described above, a tautomer, a prodrug, a pharmaceutically acceptable salt, or a solvate thereof, and one or more different type of anti-retrovirus active substances.

Further, the present invention provides a method for treating or preventing retrovirus infection which comprises a simultaneous or continuous administration of the compound represented by the formula (I), a tautomer, a prodrug, a pharmaceutically acceptable salt, or a solvate thereof, and one or more different type of anti-HIV active substances.

As another embodiment, the present invention provides use of a compound represented by the formula (I), a tautomer, a prodrug, a pharmaceutically acceptable salt, or a solvate thereof, and one or more different type of anti-retrovirus active substances for preparation of a medicament for treating or preventing retrovirus infection.

Further the present invention relates to a method for inhibiting retrovirus replication which comprises a contact of a compound represented by the formula (I), a tautomer, a prodrug, a pharmaceutically acceptable salt, or a solvate thereof, and one or more different type of anti-retrovirus active substances with retrovirus.

Furthermore, the present invention relates to a combination of a compound represented by the formula (I), a tautomer, a prodrug, a pharmaceutically acceptable salt, or a solvate thereof, and one or more different type of anti-retrovirus active substances.

That is, the present invention relates to:

(1) An anti-retrovirus composition which contains as active ingredients a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances,

(2) An anti-retrovirus composition as described in (1) wherein the anti-retrovirus active substance exhibits a synergistic effect in combination with a compound represented by the formula (I), a tautomer, a prodrug, a pharmaceutically acceptable salt, or a solvate thereof,

(3) An anti-retrovirus composition as described in (1) or (2) wherein the anti-retrovirus active substance is one excluding an integrase inhibitor,

(4) An anti-retrovirus composition as described in any one of (1) to (3) wherein the anti-retrovirus active substance is a nucleoside type reverse transcriptase inhibitor, an non-nucleoside type reverse transcriptase inhibitor and/or a protease inhibitor,

(5) An anti-retrovirus composition as described in any one of (1) to (4) wherein the anti-retrovirus active substance is zidovudine, didanosine, zalcitabine, stavudine, lamivudine, abacavir, tenofovir, tenofovir disoproxil, nevirapine, delavirdine, emivirine, loviride, efavirenz, trovirdine, capravirine, TIBO, talviraline, UC781, saquinavir, nelfinavir, ritonavir, indinavir, KNI-272, lopinavir, VX-478, VB-19026, BILA-2011-BS, A-77003, A-80987, DMP-323, and/or XM-450,

(6) An anti-retrovirus composition as described in any one of (1) to (5) wherein the anti-retrovirus active substance is zidovudine, lamivudine, stavudine, nevirapine, capravirine, and/or nelfinavir,

(7) An anti-retrovirus composition as described in any one of (1) to (6) wherein A of the compound represented by the formula (I) is optionally substituted furyl, optionally substituted thienyl, or optionally substituted pyridyl; and B of the compound represented by the formula (I) is optionally substituted triazolyl, optionally substituted tetrazolyl, optionally substituted pyridyl, or optionally substituted oxazolyl,

(8) An anti-retrovirus composition as described in any one of (1) to (7) wherein A of the compound represented by the formula (I) is 5-(4-fluorobenzyl)furan-2-yl; and B of the compound represented by the formula (I) is 1*H*-1,2,4-triazol-3-yl,

(9) An anti-retrovirus composition as described in any one of (1) to (8) wherein retrovirus is human immunodeficiency virus,

(10) A pharmaceutical composition which contains a compound, which exhibits an activity promoting the anti-retrovirus activity of an anti-retrovirus active substance, and is represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof,

(11) A method for treating or preventing retrovirus infection, which comprises a simultaneous or continuous administration of a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances,

(12) Use of a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances for preparation of a medicament for treating and preventing retrovirus infection.

## Brief Description of Drawings

Figure 1 is a graph showing a synergistic effect between a compound I-16 and zidovudine. X axis and Y axis show the concentration of medicine (ng/ml) and Z axis shows the synergistic effect ($\mu M^2\%$).

## Best Mode for Carrying Out the Invention

A compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded, exhibits inhibitory activity against retrovirus integrase (e.g., HIV, HTLV,

SIV, FIV).

Especially, the cases wherein A of the compound represented by the formula (I) is optionally substituted furyl, optionally substituted thienyl, or optionally substituted pyridyl, and B of the compound represented by the formula (I) is optionally substituted triazolyl, optionally substituted tetrazolyl, optionally substituted pyridyl, or optionally substituted oxazolyl, are preferred.

Further preferred is a compound of the formula (I) wherein A is 5-(4-fluorobenzyl)furan-2-yl, and B is 1*H*-1,2,4-triazol-3-yl.

The term "heteroaryl" includes monocyclic heteroaryl and fused heteroaryl as defined below.

The term "monocyclic heteroaryl" means a 5- to 8-membered heteroaromatic group containing 1 to 4 oxygen atom, sulfur atom and/or nitrogen atom in the ring, which may have a radical group at any substitutable position such as carbon atom or nitrogen atom.

The term "monocyclic heteroaryl" includes furyl (e.g., furan-2-yl, furan-3-yl), thienyl (e.g., thiophen-2-yl, thiophen-3-yl), pyrrolyl (e.g., pyrrol-1-yl, pyrrol-2-yl, pyrrol-3-yl), imidazolyl (e.g., imidazol-1-yl, imidazol-2-yl, imidazol-4-yl), pyrazolyl (e.g., pyrazol-1-yl, pyrazol-3-yl, pyrazol-4-yl), triazolyl (e.g., 1*H*-1,2,4-triazol-1-yl, 4*H*-1,2,4-triazol-1-yl, 1*H*-1,2,4-triazol-3-yl), tetrazolyl (e.g., 1*H*-tetrazol-1-yl, 2*H*-tetrazol-2-yl, 1*H*-tetrazol-5-yl, 2*H*-tetrazol-5-yl), oxazolyl (e.g., oxazol-2-yl, oxazol-4-yl, oxazol-5-yl), isoxazolyl (e.g., isoxazol-3-yl, isoxazol-4-yl, isoxazol-5-yl), thiazolyl (e.g., thiazol-2-yl, thiazol-4-yl, thiazol-5-yl), isothiazolyl (e.g., isothiazol-3-yl, isothiazol-4-yl, isothiazol-5-yl), pyridyl (e.g., pyridin-2-yl, pyridin-3-yl, pyridin-4-yl), pyridazinyl (e.g., pyridazin-3-yl, pyridazin-4-yl), pyrimidinyl (e.g., pyrimidin-2-yl, pyrimidin-4-yl, pyrimidin-5-yl), furazanyl (e.g., furazan-3-yl), pyrazinyl (e.g., pyrazin-2-yl), thiadiazolyl (e.g., 1,3,4-thiadiazol-2-yl), oxadiazolyl (e.g., 1,3,4-oxadiazol-2-yl) and the like.

The term "fused heteroaryl" means a heteroaromatic group wherein a 5- to 8-membered aromatic ring containing 1 to 4 oxygen atom, sulfur atom and/or nitrogen atom in the ring is fused with one to four 5- to 8-membered aromatic carbon rings or other 5- to 8-membered heteroaromatic rings, which has a radical group at any substitutable position such as carbon atom or nitrogen atom as well as monocyclic heteroaryl. The radical group may be at the heteroaromatic ring or aromatic carbon ring.

The term "fused heteroaryl" includes benzofuryl (e.g., benzo[b]furan-2-yl, benzo[b]furan-3-yl, benzo[b]furan-4-yl, benzo[b]furan-5-yl, benzo[b]furan-6-yl, benzo[b]furan-7-yl), benzothienyl (e.g., benzo[b]thiophen-2-yl, benzo[b]thiophen-3-yl, benzo[b]thiophen-4-yl, benzo[b]thiophen-5-yl, benzo[b]thiophen-6-yl, benzo[b]thiophen-7-yl), benzimidazolyl (e.g., benzimidazol-1-yl, benzimidazol-2-yl, benzimidazol-4-yl, benzimidazol-5-yl), benzothiazolyl(e.g., benzothiazol-2-yl, benzothiazol-3-yl, benzothiazol-4-yl, benzothiazol-5-yl, benzothiazol-6-yl, benzothiazol-7-yl), indolyl (e.g., indol-1-yl, indol-2-yl, indol-4-yl, indol-5-yl, indol-6-yl, indol-7-yl), dibenzofuryl, quinolinyl (e.g., quinolin-2-yl, quinolin-3-yl, quinolin-4-yl, quinolin-5-yl, quinolin-6-yl, quinolin-7-yl, quinolin-8-yl), isoquinolinyl (e.g., isoquinolin-1-yl, isoquinolin-3-yl, isoquinolin-4-yl, isoquinolin-5-yl, isoquinolin-6-yl, isoquinolin-7-yl, isoquinolin-8-yl), cinnolinyl (e.g., cinnolin-3-yl, cinnolin-4-yl, cinnolin-5-yl, cinnolin-6-yl, cinnolin-7-yl, cinnolin-8-yl), quinazolinyl (e.g., quinazolin-2-yl, quinazolin-4-yl, quinazolin-5-yl, quinazolin-6-yl, quinazolin-7-yl, quinazolin-8-yl), quinoxalinyl (e.g., quinoxalin-2-yl, quinoxalin-5-yl, quinoxalin-6-yl), phthalazinyl (e.g., phthalazin-1-yl, phthalazin-5-yl, phthalazin-6-yl), purinyl (e.g., purin-2-yl, purin-6-yl, purin-7-yl, purin-8-yl, purin-9-yl), pteridinyl, carbazolyl, phenanthridinyl, acridinyl, phenazinyl, 1,10-phenanthrolinyl, isoindolyl, 1H-indazolyl, indolidinyl (e.g., indolidin-1-yl) or the like.

The term "aryl" means a monocyclic aromatic hydrocarbon group like phenyl or a polycyclic aromatic hydrocarbon group such as naphthyl, phenanthryl and the like. Preferred is phenyl or naphthyl.

When "aryl" or "heteroaryl" has a substituent(s), one to four, same or different substituent(s) may be at any substitutable position(s).

Examples of the substituent include hydroxy, carboxy, halogen (F, Cl, Br, I), lower haloalkyl (e.g., $CF_3$, $CH_2CF_3$), lower alkyl (e.g., methyl, ethyl, isopropyl, tert-butyl), lower alkenyl (e.g., vinyl, allyl), lower alkynyl (e.g., ethynyl), cycloalkyl (e.g., cyclopropyl, cyclobutyl, cyclohexyl), cycloalkenyl (e.g., 1-cyclohexenyl), lower alkyloxy (e.g., methoxy, ethoxy, propoxy, butoxy), lower alkyloxycarbonyl (e.g., methoxycarbonyl, ethoxycarbonyl, tert-butoxylcarbonyl), nitro, nitroso, amino, amino substituted with lower alkyl (e.g., methylamino, ethylamino, dimethylamino), azido, amidino, guanidino, optionally substituted aryl (e.g., phenyl, p-tolyl), heteroaryl (e.g., pyridyl, furyl), heteroaryl(lower)alkyl (e.g., picolyl), optionally substituted aryl(lower)alkyl (e.g., benzyl, 4-methylbenzyl, 4-fluorobenzyl), aryl(lower)alkyloxy (e.g., benzyloxy), aryl(lower)alkylthio (e.g., benzylthio), cyano, isocyano, hydroxylamino, mercapto, lower alkylthio (e.g., methylthio), carbamoyl, carbamoyl substituted with lower alkyl (e.g., N-methylcarbamoyl), lower alkylsulfonyl (e.g., mesyl, ethanesulfonyl), optionally substituted arylsulfonyl (e.g., benzenesulfonyl, 2-toluenesulfonyl, 4-toluenesulfonyl), sulfamoyl, sulfoamino, formyl, lower alkylcarbonyl (e.g., acetyl, propionyl, benzoyl, p-toluoyl, cyclohexylcarbonyl), lower alkylcarbonyloxy (e.g., acetyloxy, benzoyloxy), hydrazino, arylamino (e.g., anilino, toluidino, xylidino), lower alkylcarbonylamino (e.g., acetamido), arylcarbonylamino (e.g., benzamido), optionally substituted arylthio (e.g., phenylthio), morpholino, morpholinyl (e.g. 2-morpholinyl, 3-morpholinyl), a group of the formula: $-Z^1-Z^2-Z^3-R^1$ wherein $Z^1$ and $Z^3$ are each independently a single bond, lower alkylene, or lower alkenylene; $Z^2$ is a single bond, lower alkylene, lower alkenylene, -CH(OH)-, -S-, -SO-, $-SO_2-$, $-SO_2NH-$, $-NHSO_2-$, -O-, -NH-, -NHCO-, -CONH-, -C(=O)-O-, -O-C(=O)-, or

-CO-; $R^1$ is optionally substituted aryl, optionally substituted heteroaryl, optionally substituted cycloalkyl, optionally substituted cycloalkenyl, or optionally substituted heterocyclic group, and the like.

The term "lower alkylene" means a C1-C6 straight or branched alkylene group, for example, methylene, ethylene, trimethylene, propylene, tetramethylene, ethylethylene, pentamethylene, hexamethylene or the like. Preferred is a C1-C4 straight alkylene group such as methylene, ethylene, trimethylene or tetramethylene.

The term "lower alkenylene" means a C2-C6 straight or branched alkenylene group which is the above "lower alkylene" having one or more double bond, for example, vinylene, propenylene, butenylene or the like. Preferred is a C2-3 straight alkenylene group such as vinylene or propenylene.

The term "cycloalkyl" means a C3-C8 cyclic alkyl group, for example, cyclopropyl, cyclobutyl, cyclopentyl, cyclohexyl, cycloheptyl, cyclooctyl or the like. Preferred is a C3-C6 cyclic alkyl group such as cyclopropyl, cyclobutyl, cyclopentyl or cyclohexyl.

The term "cycloalkenyl" means a C3-C8 cyclic alkenyl group which is the above "cycloalkyl" having one or more double bond, for example, 1-cyclopropen-1-yl, 2-cyclopropen-1-yl, 1-cyclobuten-1-yl, 2-cyclobuten-1-yl, 1-cyclopenten-1-yl, 2-cyclopenten-1-yl, 3-cyclopenten-1-yl, 1-cyclohexen-1-yl, 2-cyclohexen-1-yl, 3-cyclohexen-1-yl, 1-cyclohepten-1-yl, 2-cyclohepten-1-yl, 3-cyclohepten-1-yl, 4-cyclohepten-1-yl or the like. Preferred is a C3-C6 cyclic alkenyl group, for example, 1-cyclopropen-1-yl, 2-cyclopropen-1-yl, 1-cyclobuten-1-yl, 2-cyclobuten-1-yl, 1-cyclopenten-1-yl, 2-cyclopenten-1-yl, 3-cyclopenten-1-yl, 1-cyclohexen-1-yl, 2-cyclohexen-1-yl or 3-cyclohexen-1-yl.

The term "heterocycle" means a non-aromatic group which is the above "cycloalkyl" or "cycloalkenyl" having 1 to 3 oxygen atom, sulfur atom and/or nitrogen atom in the ring, for example, aziridinyl (e.g., aziridin-1-yl, aziridin-2-yl), piperidino, piperidyl (e.g., 2-piperidyl, 3-piperidyl, 4-piperidyl), morpholino, morpholinyl (e.g., 2-morpholinyl, 3-morpholinyl), pyrrolinyl (e.g., 1-pyrrolinyl, 2-pyrrolinyl, 3-pyrrolinyl, 4-pyrrolinyl, 5-pyrrolinyl), pyrrolidinyl (e.g., 1-pyrrolidinyl, 2-pyrrolidinyl, 3-pyrrolidinyl), imidazolinyl (e.g., 1-imidazolinyl, 2-imidazolinyl, 4-imidazolinyl), piperazino, piperazinyl (e.g., 2-piperazinyl), thiolanyl (e.g., thiolan-2-yl, thiolan-3-yl), tetrahydrofuranyl (e.g., tetrahydrofuran-2-yl, tetrahydrofuran-3-yl), dioxanyl (e.g., 1,4-dioxan-2-yl), oxathianyl (e.g., 1,4-oxathian-2-yl, 1,4-oxathian-3-yl), tetrahydropyranyl (e.g., tetrahydropyran-2-yl, tetrahydropyran-3-yl, tetrahydropyran-4-yl) or the like. Preferred is a 5- or 6-membered N- containing heterocycle such as piperidino, piperidyl (e.g., 2-piperidyl, 3-piperidyl, 4-piperidyl), morpholino, morpholinyl (e.g., 2-morpholinyl, 3-morpholinyl), pyrrolinyl (e.g., 1-pyrrolinyl, 2-pyrrolinyl, 3-pyrrolinyl, 4-pyrrolinyl, 5-pyrrolinyl), pyrrolidinyl (e.g., 1-pyrrolidinyl, 2-pyrrolidinyl, 3-pyrrolidinyl), imidazolinyl (e.g., 1-imidazolinyl, 2-imidazolinyl, 4-imidazolinyl), piperazino, piperazinyl (e.g., 2-piperazinyl), and the like.

For example, a compound represented by the formula (I) of A-C(=O)-CH=C(OH)-B wherein A and B are as defined above, is a compound described in PCT/JP99/07101. More preferred are the following compounds.

(I-1) 1-[1*H*-1-(4-Fluorobenzyl)pyrazol-4-yl]-3-hydroxy-3-(1*H*-1,2,4-triazol-3-yl)propenone,
(I-2) 1-[4-(4-Fluorobenzyl)furan-2-yl]-3-hydroxy-3-(1*H*-1,2,4-triazol-3-yl)propenone,
(I-3) 1-[5-(4-Fluorobenzyl)furan-2-yl]-3-hydroxy-3-(2*H*-tetrazol-5-yl)propenone,
(I-4) 3-Hydroxy-1-(5-phenylthiofuran-2-yl)-3-(1*H*-1,2,4-triazol-3-yl)propenone,
(I-5) 1-(5-Benzenesulfonylfuran-2-yl)-3-hydroxy-3-(1*H*-1,2,4-triazol-3-yl)propenone,
(I-6) 1-(5-n-Butylfuran-2-yl)-3-hydroxy-3-(1*H*-1,2,4-triazol-3-yl)propenone,
(I-7) 1-[5-(4-Fluorobenzyl)thiophen-2-yl]-3-hydroxy-3-(1*H*-1,2,4-triazol-3-yl)propenone,
(I-8) 1-(5-n-Butylfuran-2-yl)-3-hydroxy-3-(2*H*-tetrazol-5-yl)propenone,
(I-9) 1-[5-(4-Fluorobenzyl)furan-3-yl]-3-hydroxy-3-(1*H*-1,2,4-triazol-3-yl)propenone,
(I-10) 1-[5-(4-Fluorobenzyl)pyrrol-2-yl]-3-hydroxy-3-(1*H*-1,2,4-triazol-3-yl)propenone,
(I-11) 1-[3-(4-Fluorobenzyl)furan-2-yl]-3-hydroxy-3-(2*H*-tetrazol-5-yl)propenone,
(I-12) 1-[3-(4-Fluorobenzyl)furan-2-yl]-3-hydroxy-3-(1*H*-1,2,4-triazol-3-yl)propenone,
(I-13) 1-[4-(4-Fluorobenzyl)furan-3-yl]-3-hydroxy-3-(1*H*-1,2,4-triazol-3-yl)propenone,
(I-14) 1-[2*H*-2-(4-Fluorobenzyl)pyrazol-3-yl]-3-hydroxy-3-(2*H*-tetrazol-5-yl)propenone,
(I-15) 1-[[4-(4-Fluorobenzyl)-1-methoxymethyl]pyrrol-3-yl]-3-hydroxy-3-(1*H*-1,2,4-triazol-3-yl)propenone,
(I-16) 1-[5-(4-Fluorobenzyl)furan-2-yl]-3-hydroxy-3-(1*H*-1,2,4-triazol-3-yl)propenone,
(I-17) 1-[[4-(4-Fluorobenzyl)-1-propyl]pyrrol-3-yl]-3-hydroxy-3-(1*H*-1,2,4-triazol-3-yl)propenone,
(I-18) 1-[1,4-Di-(4-fluorobenzyl)pyrrol-3-yl]-3-hydroxy-3-(1*H*-1,2,4-triazol-3-yl)propenone,
(I-19) 1-[4-(4-fluorobenzyl)pyrrol-3-yl]-3-hydroxy-3-(1*H*-1,2,4-triazol-3-yl)propenone,
(I-20) 1-[2-(4-Fluorobenzyl)furan-3-yl]-3-hydroxy-3-(2*H*-tetrazol-5-yl)propenone,
(I-21) 1-[[1-Benzenesulfonyl-4-(2-phenylethyl)]pyrrol-3-yl]-3-hydroxy-3-(2*H*-tetrazol-5-yl)propenone,
(I-22) 3-Hydroxy-1-[(4-(2-phenylethyl))pyrrol-3-yl]-3-(2*H*-tetrazol-5-yl)propenone,
(I-23) 1-[[1-Benzyl-4-(2-carboxyvinyl)]pyrrol-2-yl]-3-hydroxy-3-(1*H*-1,2,4-triazol-3-yl)propenone,
(I-24) 1-[[1-Benzyl-4-(2-carboxyvinyl)]pyrrol-3-yl]-3-hydroxy-3-(1*H*-1,2,4-triazol-3-yl)propenone,
(I-25) 1-[2-(4-Fluorobenzyl)furan-3-yl]-3-hydroxy-3-(1*H*-1,2,4-triazol-3-yl)propenone,
(I-26) 1-[1-(4-Fluorobenzyl)pyrrol-3-yl]-3-hydroxy-3-(2*H*-tetrazol-5-yl)propenone,

(I-27) 1-[2-(4-Fluorobenzyl)benzothiophen-3-yl]-3-hydroxy-3-(1*H*-1,2,4-triazol-3-yl)propenone,
(I-28) 1-[2-(4-Fluorobenzyl)benzofuran-3-yl]-3-hydroxy-3-(1*H*-1,2,4-triazol-3-yl)propenone,
(I-29) 1-[(1-Benzyl-5-carboxy)pyrrol-3-yl]-3-hydroxy-3-(1*H*-1,2,4-triazol-3-yl)propenone,
(I-30) 1-[(1-Benzyl-5-ethoxycarbonyl)pyrrol-3-yl]-3-hydroxy-3-(1*H*-1,2,4-triazol-3-yl)propenone,
(I-31) 1-[[1-Benzyl-5-(2-carboxyvinyl)]pyrrol-3-yl]-3-hydroxy-3-(1*H*-1,2,4-triazol-3-yl)propenone,
(I-32) 1-[1-(4-Fluorobenzyl)pyrrol-2-yl]-3-hydroxy-3-(1*H*-1,2,4-triazol-3-yl)propenone,
(I-33) 1-[1-(4-Fluorobenzyl)pyrrol-2-yl]-3-hydroxy-3-(2*H*-tetrazol-5-yl)propenone,
(I-34) 1-(1-Benzenesulfonylpyrrol-3-yl)-3-hydroxy-3-(1*H*-1,2,4-triazol-3-yl)propenone,
(I-35) 1-[2-(4-Fluorobenzyl)benzofuran-3-yl]-3-hydroxy-3-(2*H*-tetrazol-5-yl)propenone,
(I-36) 1-(2-Benzylbenzofuran-3-yl)-3-hydroxy-3-(2*H*-tetrazol-5-yl)propenone,
(I-37) 1-[(1-Benzenesulfonyl-4-ethyl)pyrrol-3-yl]-3-hydroxy-3-(2*H*-tetrazol-5-yl)propenone,
(I-38) 1-(1-Benzylpyrrol-3-yl)-3-hydroxy-3-(1*H*-1,2,4-triazol-3-yl)propenone,
(I-39) 1-[[1-Benzyl-5-(2-methoxycarbonylethyl)]pyrrol-3-yl]-3-hydroxy-3-(2*H*-tetrazol-5-yl)propenone,
(I-40) 1-[[1-Benzyl-5-(2-methoxycarbonylvinyl)]pyrrol-3-yl]-3-hydroxy-3-(2*H*-tetrazol-5-yl)propenone,
(I-41) 1-[(1-Benzyl-5-ethoxycarbonyl)pyrrol-3-yl]-3-hydroxy-3-(2*H*-tetrazol-5-yl)propenone,
(I-42) 1-[(1-Benzyl-5-n-butyl)pyrrol-3-yl]-3-hydroxy-3-(2*H*-tetrazol-5-yl)propenone,
(I-43) 1-[(1-Benzyl-5-n-propyl)pyrrol-3-yl]-3-hydroxy-3-(2*H*-tetrazol-5-yl)propenone,
(I-44) 1-(1-Benzylpyrrol-3-yl)-3-hydroxy-3-(2*H*-tetrazol-5-yl)propenone,
(I-45) 1-(1-Benzenesulfonylpyrrol-3-yl)-3-hydroxy-3-(2*H*-tetrazol-5-yl)propenone,
(I-46) 1-[5-(4-Fluorobenzyl)furan-2-yl]-3-hydroxy-3-(pyridin-2-yl)propenone,
(I-47) 1-[5-(4-Fluorobenzyl)furan-2-yl]-3-hydroxy-3-(pyrimidin-2-yl)propenone,
(I-48) 3-(5-Carboxypyridin-2-yl)-1-[5-(4-fluorobenzyl)furan-2-yl]-3-hydroxypropenone,
(I-49) 3-(4-Carboxypyridin-2-yl)-1-[5-(4-fluorobenzyl)furan-2-yl]-3-hydroxypropenone,
(I-50) 1-[2-(4-Fluorobenzyl)oxazol-5-yl]-3-hydroxy-3-(pyridin-2-yl)propenone,
(I-51) 1- [2-(4-Fluorobenzyl)oxazol-5-yl]-3-hydroxy-3-(pyrimidin-2-yl)propenone.

Especially, preferred is (I-16) 1- [5-(4-Fluorobenzyl)furan-2-yl]-3-hydroxy-3-(1*H*-1,2,4-triazol-3-yl)propenone.

A compound represented by the formula (I) can be in any chemical structure of E form and Z form on the substituent represented by the formula of -CH=C(OH)-, both of which are included in a compound represented by the formula (I). And a tautomer of a compound represented by the formula (I) can be used. That is, a compound of any chemical structure shown below can be used.

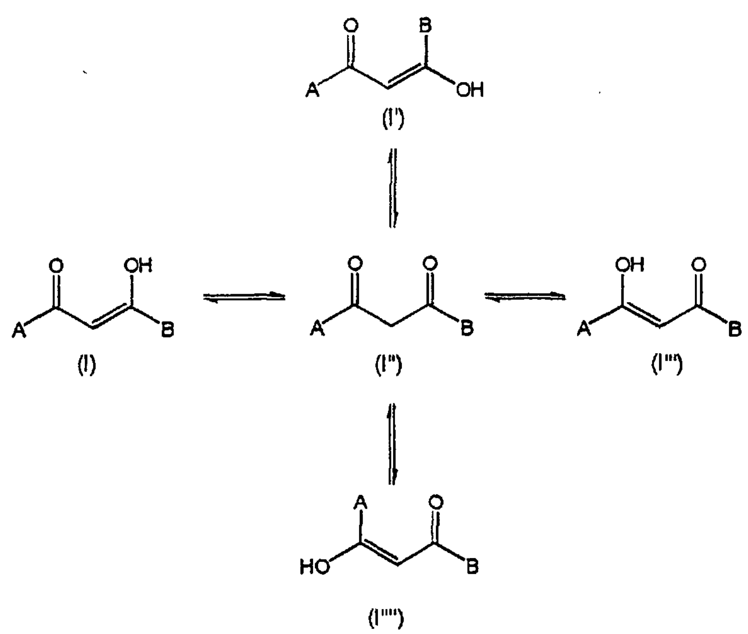

(I')

(I) (I") (I''')

(I'''')

Prodrug means a compound which can be converted into a compound represented by the formula (I) in vivo. For example, when the compound represented by the formula (I) has an amino group or a hydroxy group, prodrug means the compound protected by an acyl group (formyl, acetyl, propionyl, butyryl, isobutyryl, valeryl, isovaleryl, pivaloyl, hexanoyl, octanoyl, lauroyl, palmitoly, stearoyl, oleoyl, acryloyl, metaacryloyl, chloroformyl, pyruvoyl, oxalo, methoxalyl, ethoxalyl, cyclohexylcarbonyl, benzoyl, o-toluoyl, m-toluoyl, p-toluoyl, cinnamoyl, 1-naphthoyl, 2-naphthoyl, salicyloyl, anisoyl, e. g.). And when the compound represented by the formula (I) has carboxylic group, prodrug means the compound converted to its ester derivative (methyl ester, ethyl ester, benzyl ester, e.g.) or its amide derivative (methylaminocarbonyl, benzylaminocarbonyl, e.g.).

Examples of a pharmaceutical acceptable salt of the compound represented by the formula (I) are a salt of mineral acid such as hydrochloric acid, sulfuric acid, nitric acid, phosphoric acid, hydrofluoric acid, hydrobromic acid, and a salt of organic acid such as formic acid, acetic acid, tartaric acid, lactic acid, citric acid, fumaric acid, maleic acid, succinic acid, methansulfonic acid, ethansulfonic acid, benzenesulfonic acid, toluenesulfonic acid, naphthalenesulfonic acid, camphorsulfonic acid, salt of alkali metal such as sodium, potassium, calcium, or salt of alkaline-earth metal.

Examples of a solvate of the compound represented by the formula (I) are hydrate (monohydrate, dihydrate).

"Anti-retrovirus active substance" employed in the present invention means any medicine used for preventing or treating retrovirus infection, including that not directly acting on retrovirus. The examples include inhibitor against enzyme derived from host (e.g., DNA polymerase inhibitor), inhibitor against entry of virus into host cell (e.g., peptide analogue such as gp120, gp41), antagonist of receptor bound by virus in host (e.g., CCR5 antagonist), chemotherapy agent, immunomodulator agent, and the like. Further included are medicines used for preventing or treating opportunistic infection and treating agent against cytomegalovirus amphiblestritis, which is a foscarnet having a DNA polymerase inhibitory effect and a reverse transcriptase inhibitory effect.

Especially, preferred is a substance having an anti-retrovirus activity as an "anti-retrovirus active substance", and more preferred is a substance directly acting on retrovirus. Examples of anti-retrovirus active substance include CCR5 antagonist, nucleoside type reverse transcriptase inhibitor, nucleoside type reverse transcriptase inhibitor, integrase inhibitor, protease inhibitor and the like, and examples of a substance directly acting on retrovirus include nucleoside type reverse transcriptase inhibitor, nucleoside type reverse transcriptase inhibitor, integrase inhibitor, and protease inhibitor.

Furthermore, as an anti-retrovirus active substance employed in the present invention, prefferd is one having a different action mechanism from a compound represented by the formula (I), such as CCR5 antagonist, nucleoside type reverse transcriptase inhibitor, nucleoside type reverse transcriptase inhibitor, and protease inhibitor.

Furthermore, an anti-retrovirus active substance employed in the present invention means, not limiting thereto, any substance which can be evaluated as having an anti-retrovirus activity by the assay therefor. For example, an anti-human immunodeficiency virus active substance can be decided by such a MTT assay method as described in reference example 2 in the present specification. Especially, preferred is anti-human immunodeficiency virus active substance which has an $EC_{50}$ value of 10 or less μmol/ml by MTT assay method.

Preferable anti-retrovirus active substances are described concretely as follows, which are not to be constructed as limiting these substances.

Example of CCR5 antagonism is TAK-779.

TAK-779 is represented by the formula:

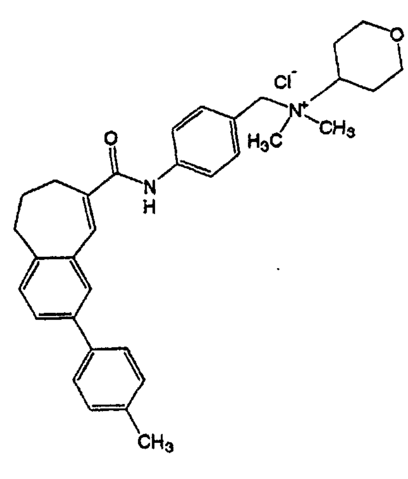

and disclosed in WO99/33976.

Example of a nucleoside type reverse transcriptase inhibitor are zidovudine (AZT), didanosine (ddI), zalcitabine (ddC), stavudine (d4T), lamivudine (3TC), abacavir (ABC), tenofovir, tenofovir disoproxil, and the like.

Zidovudine is represented by the formula:

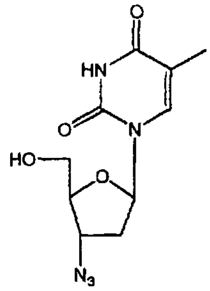

its chemical name is 3'-azido-3'-deoxycytimidine. Zidovudine can be prepared and used by the method described in US4,724,232.

Didanosine is represented by the formula:

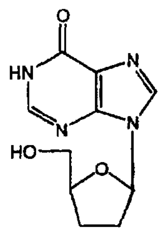

its chemical name is 2',3'-dideoxyinosine. Didanosine is disclosed in US4,861,759 and WO87/01283.

Zalcitabine is represented by the formula:

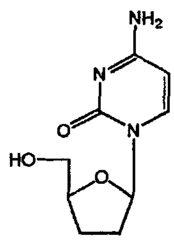

its chemical name is 2',3'-dideoxycytidine. Zalcitabine is disclosed in US4,879,277 and WO87/01283.

Stavudine is represented by the formula:

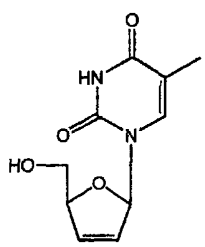

Stavudine is disclosed in EP398230.

Lamivudine is represented by the formula:

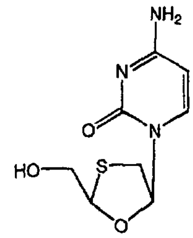

its chemical name is (-)-2',3'-dideoxy-3'-thiacytidine. Lamivudine is disclosed in EP0,382,526.

Abacavir is represented by the formula:

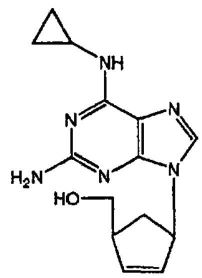

Abacavir is being developed as its sulfate. In the present invention Abacavir can be used as a pharmaceutical acceptable salt, mainly various acid salts, and preferably sulfuric acid salt. Abacavir is disclosed in EP434450.

Tenofovir is represented by the formula:

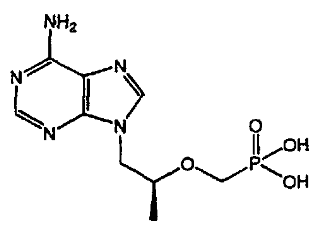

its chemical name is (R)-9-(2-phosphonylmethoxypropyl)adenine. Tenofovir is disclosed in US5,733,788 and WO94/03467.

Tenofovir disoproxil is represented by the formula:

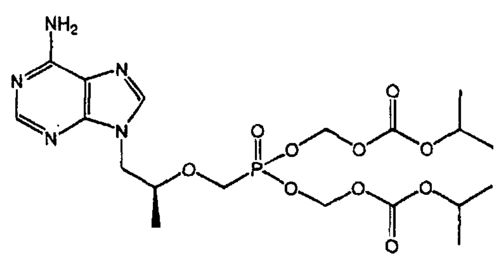

its chemical name is (R)-[[2-(6-amino-9H-purin-9-yl)-1-methylethoxy]methyl]phosphonic bis(isopropoxycarbonyloxymethyl) ester. Tenofovir disoproxil is being developed as its fumaric acid salt. In the present invention tenofovir disoproxil can be used as a pharmaceutical acceptable salt, mainly various acid salts, preferably fumaric acid salt. Tenofovir is disclosed in US5,922,695.

Examples of non-nucleoside type reverse transcriptase inhibitor are nevirapine, delavirdine, emivirine, loviride, efavirenz, trovirdine, capravirine, TIBO, talviraline, UC781, and the like.

Nevirapine is represented by the formula:

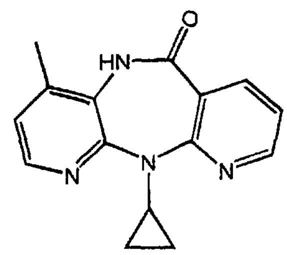

Nevirapine is disclosed in EP429987.

Delavirdine is represented by the formula:

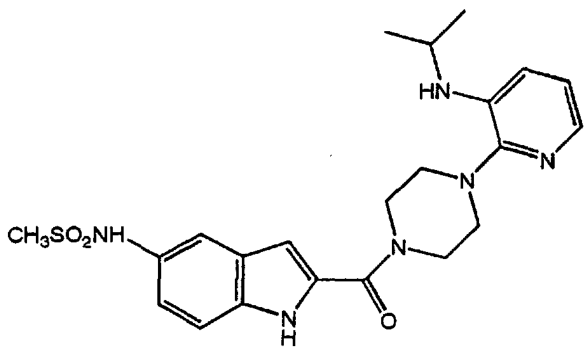

its chamical name is 1-[5-methanesulfonamideindolyl-2-carbonyl]-4-[3-(1-methylethylamino)-2-pyridyl]piperadine. Delavirdine is used as a non-nucleoside type reverse transcriptase inhibitor named "RESCRIPTOR" sold by Upjohn. In the present invention delavirdine can be used as a pharmaceutical acceptable salt, mainly various acid salts, and preferably methanesulfonic acid salt.

Emivirine is represented by the formula:

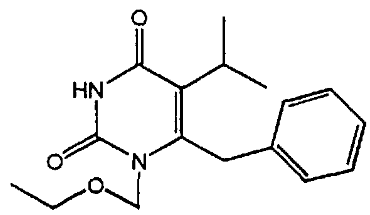

its chemical name is 6-benzyl-1-(ethoxymethyl)-5-isopropyluracil. Emivirine is disclosed in EP420763.

Loviride is represented by the formula:

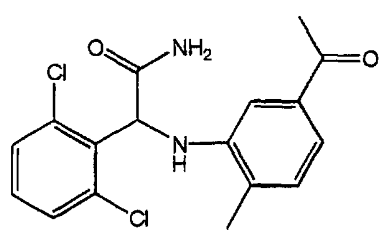

its chemical name is (±)-α-[(2-acetyl-5-methylphenyl)amino]-2,6-dichlorobenzeneacetomide. Loviride is disclosed in EP0,538,301.

Efavirenz is represented by the formula:

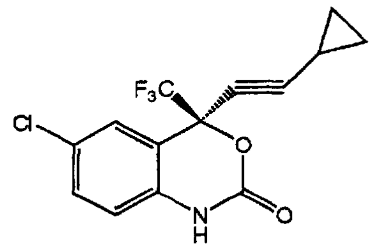

its chemical name is (S)-(-)-6-chloro-4-(chloropropylethynyl)-1,4-dihydro-4-(trifluoromethyl)-2H-3,1-benzoxazin-2-one. Efavirenz is disclosed in WO94/03440 and EP582455.

Trovirdine is represented by the formula:

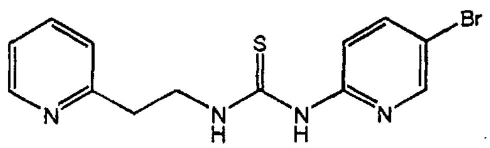

its chemical name is 1-(5-bromo-2-pyridyl)-3-[2-(2-pyridyl)ethyl]-2-thiourea, In the present invention trovirdine can be used as a pharmaceutical acceptable salt, mainly various acid salts, and preferably hydrochloric acid salt. Trovirdine is disclosed in WO93/03022.

Capravirine is represented by the formula:

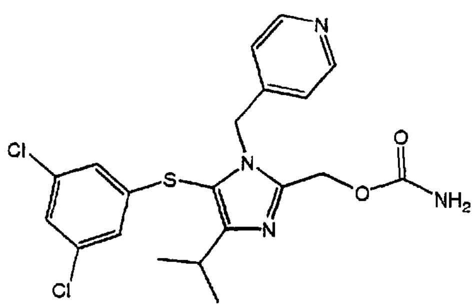

its chemical name is 5-[(3,5-dichlorophenyl)thio]-4-(1-methylethyl)-1-(4-pyridynylmethyl)-1H-imidazole-2-methanol carbamate. In the present invention capravirine can be used as a pharmaceutical acceptable salt, mainly various acid salts, and preferably hydrochloric acid salt. Capravirine is disclosed in WO96/10019.

TIBO is represented by the formula:

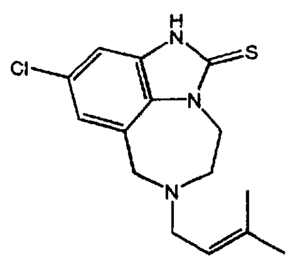

its chemical name is (+)-S-4,5,6,7-tetrahydro-S-methyl-6-(3-methyl-2-butenyl)-imidazo(4,5,1-jk)(1,4)-benzodiazepin-2(1H)-thione. TIBO is disclosed in EP384522.

Talviraline (HBY097) is represented by the formula:

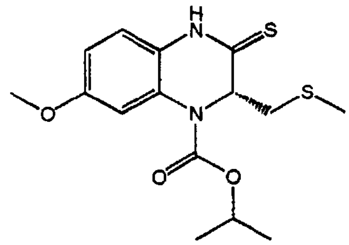

Talviraline (HBY097) is disclosed in EP509398.

UC781 is represented by the formula:

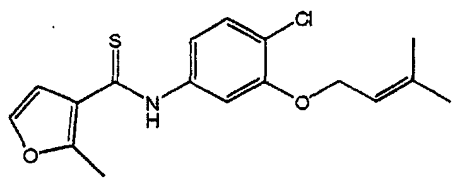

UC781 is disclosed in WO97/19940.

Examples of HIV protease inhibitor are saquinavir, nelfinavir, ritonavir, indinavir, KNI-272, lopinavir, VX-478, VB-19026, BILA-2011-BS, A-77003, A-80907, DMP-323, XM-450 and the like.

Saquinavir is represented by the formula:

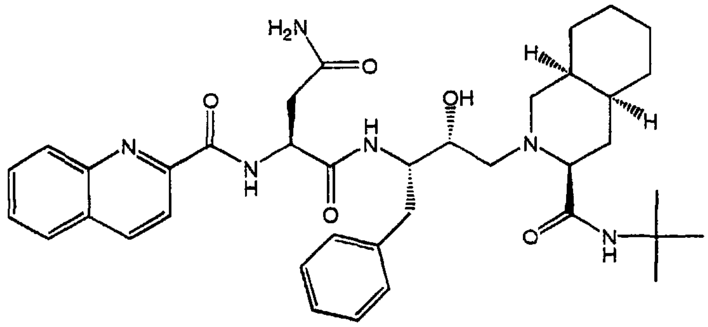

its chemical name is N-tert-butyl-decahydro-2-[2(R)-hydroxy-4-phenyl-3(S)-{[N-(2-quinolylcarbonyl)-L-asparginyl]amino}butyl]-(4aS,8aS)-isoquinoline-3(S)-carboxamide. Saquinavir's methanesulfonic acid salt $C_{30}H_{50}N_6O_5$ $CH_4O_3S$ (molecular weight 766.96) is sold by Roche as HIV protease inhibitor named "INVIRASW" for oral administration. In the present invention Saquinavir can be used as a pharmaceutical acceptable salt, mainly various acid salts, and preferably methanesulfonic acid salt. Saquinavir is disclosed in US5,196,438.

Nelfinavir is represented by the formula:

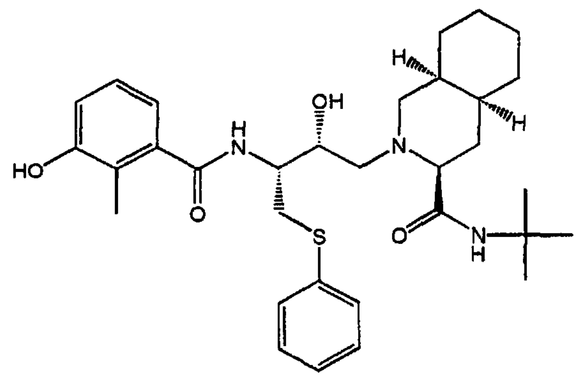

its chemical name is [3S-[2(2S*,3S*),3α,4aβ,8aβ]]-N-(1,1-dimethylethyl)-decahydro-2-[2-hydroxy-3-[(3-hydroxy-2-methylbenzoyl)amino]-4-(phenylthio)butyl]-3-isoquinolinecarboxamide. Nelfinavir's methanesulfonic acid salt (molecular weight (663.90) is sold by Agron as HIV protease inhibitor named "VIRACEPT" for oral administration. In the present invention nelfinavir can be used as a pharmaceutical acceptable salt, mainly various acid salts, and preferably methanesulfonic acid salt. Nelfinavir is disclosed in WO95/09843 and US5,484,926.

Ritonavir is represented by the formula:

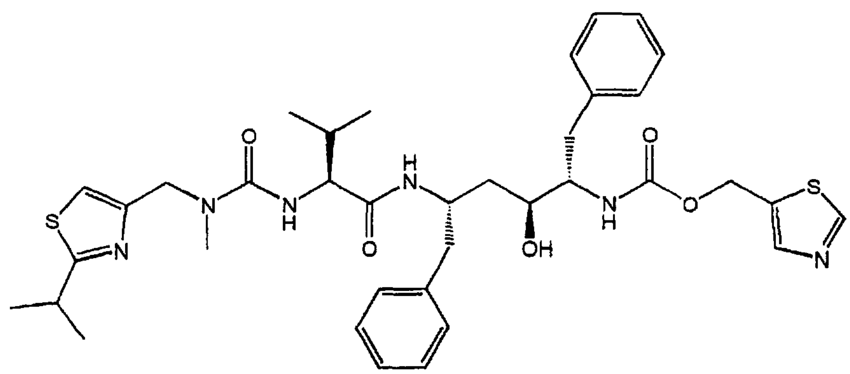

its chemical name is [5S-(5R*,8R*10R*,11R*)]-10-hydroxy-2-methyl-5-(1-methylethyl)-1-[2-(1-methylethyl)-4-thiazolyl]-3,6-dioxo-8,10-bis(phenylmethyl)-2,4,7,12-tetraazatridecane-13-hydroxy acid 5-thiazolylmethyl ester (chemical formula $C_{37}H_{48}N_6O_5S_2$, molecular weight 720.95). Ritonavir's citric acid salt is sold by Abott. as HIV protease inhibitor named "NORVIR" for oral administration. In the present invention ritonavir can be used as a pharmaceutical acceptable salt, mainly various acid salt, and preferably citric acid salt. Ritonavir is synthesized by the method disclosed in WO94/14436.

Indinavir is represented by the formula:

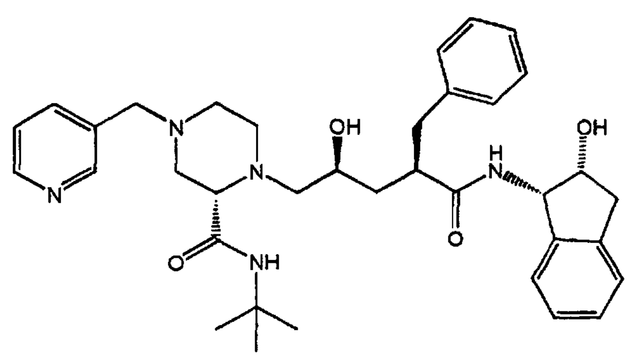

its chemical name is [1(1S,2R),5(s)]-2,3,5-trideoxy-N-(2,3-dihydro-2-hydroxy-1H-inden-1-yl)-5-[2-{[(1,1-dimethylethyl) amino]carbonyl}-4-(3-pyridinylmethyl)-1-piperadinyl]-2-(phenylmethyl)-D-erythro-pentonamide. Indinavir's sulfuric ac-

id salt ($C_{36}H_{47}N_5O_4$ $H_2SO_4$, molecular weight 711.88) is sold by Merck as HIV protease inhibitor named "CRIXIVAN" for oral administration. In the present invention ritonavir can be used as a pharmaceutical acceptable salt, mainly a various acid salt, preferably sulfuric acid salt. Indinavir is disclosed in EP541168 and US5,413,999.

KNI-272 is represented by the formula:

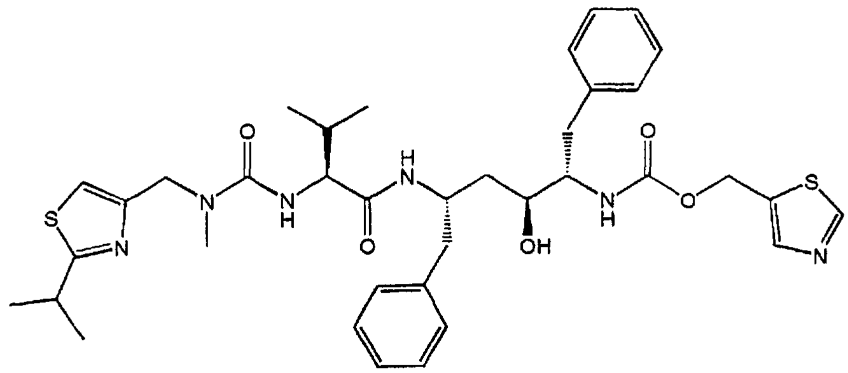

its chemical name is (R)-N-tert-2,3,5-butyl-3-[(2S,3S)-2-hydroxy-3-[(R)-2-(5-isoquinolyloxyacetyl)amino-3-methylthiopropanoyl]amino-4-phenylbutanoyl]-1,3-thiazolidine-4-carboxamide. In the present invention KNI-272 can be used as a pharmaceutical acceptable salt, mainly various acid salts. KNI-272 is disclosed in EP574135.

Lopinavir (ABT-378) is represented by the formula:

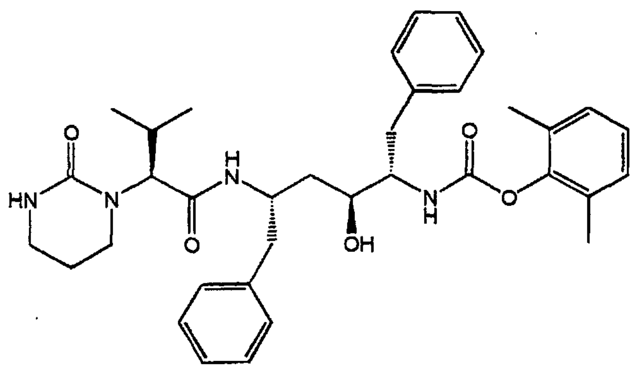

In the present invention lopinavir can be used as a pharmaceutical acceptable salt, mainly various acid salts. Lopinavir is disclosed in WO97/21685.

VX-478 is represented by the formula:

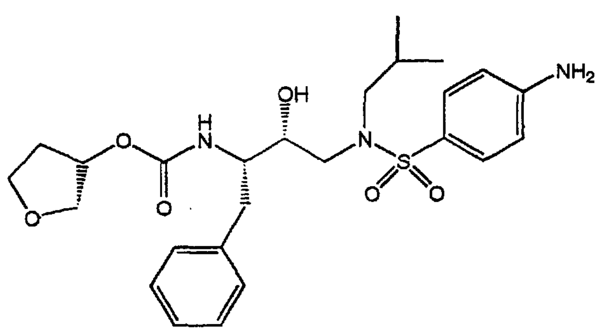

it is disclosed in WO94/05639. In the present invention VX-478 can be used as a pharmaceutical acceptable salt, mainly various acid salts.

VB-19026 is represented by the formula:

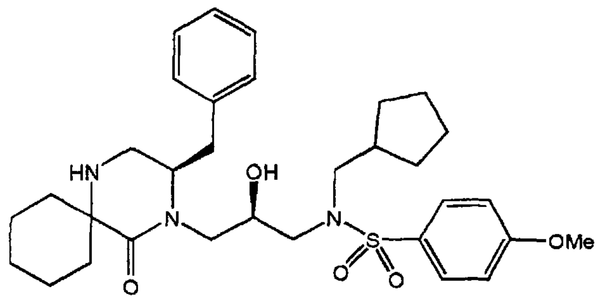

VB-19026 is disclosed in WO97/27180.

BILA-2011-BS is represented by the formula:

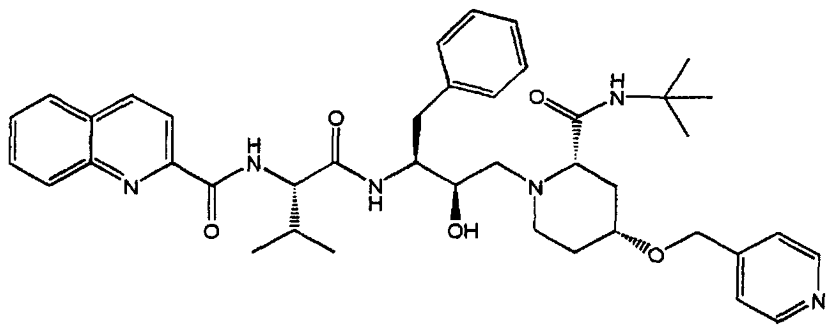

it is disclosed in EP560268.

A-77003 is (2S,3R,4S,5S)-2,5-di-(N-(N-methyl)-N-(2-pyridyl)methyl)amino)-carbonylvalinylamino)-3,4-dihydroxy-1,6-diphenylhexane. In the present invention A-77003 can be used as a pharmaceutical acceptable salt, mainly various acid salts. A-77003 is disclosed in US5,142,056.

A-80987 is (2S,3R,5S)-2-(N-(N-((2-pyridinyl)methoxycarbonyl)valinyl)amino)-5-(N-(3-pyridinyl)methoxycarbonyl)amino)-1,6-diphenyl-3-hydroxyhexane. In the present invention A-80987 can be used as a pharmaceutical acceptable salt, mainly various acid salts. A-80987 is disclosed in US5,354,866.

DMP-323 is represented by the formula:

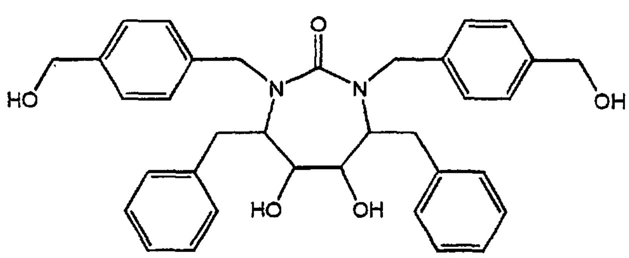

it is disclosed in WO93/07128.

XM-450 is represented by the formula:

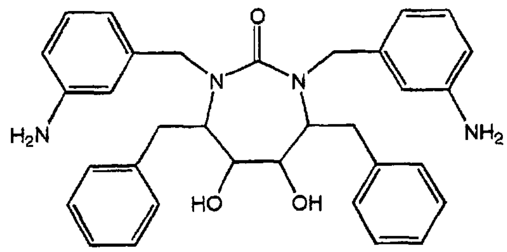

it is disclosed in WO93/071

In compounds described above, especially zidovudine, lamivudine, stavudine, nevirapine, capravirine and nelfinavir are preferable.

These anti-retrovirus active substances exhibit an synergistic effect of anti-retrovirus activity, especially anti-HIV activity, in combination with a compound represented the formula (I).

Retrovirus means a virus having reverse transcriptase, including human immunodeficiency virus (e.g., HIV-1, HIV-2), human T cell leukemic virus (e.g., HTLV-I, HTLV-II), feline immunodeficiency virus (e.g., FIV), simian immunodeficiency virus (e.g., SIV), and the like.

These virus have reverse transcriptase, integrase, and protease, each of their reverse transcriptase, integrase, and protease has high homology because their function are same.

Therefore, the anti-retrovirus compositions of the present invention can inhibit the production of various retroviruses, for treating or preventing retro-virus infection.

For example, a compound represented by the formula (I) can inhibit integrase of not only human immunodeficiency virus (HIV), but also other retro-viruses.

Anti-retrovirus compositions of the present invention can effectively treat or prevent retro-virus infection because the compositions exhibit the synergistic effect compared with single administration of each anti-retrovirus active substance.

The anti-retrovirus effect can fully obtained by even a smaller dose of each anti-retrovirus active substance of the composition than each single dose concretely without a side-effect such as toxicity, thanks to their synergistic effect as shown in below mentioned examples.

In addition, the same amount of each anti-retrovirus active substance of the composition as each single dose, can excellently prevent the production of medicine-resistant retrovirus, allowing powerful and useful treatment.

As above mentioned, anti-retrovirus compositions of the present invention are very useful pharmaceutical compositions for retrovirus infection, especially treating agent and preventing agent for AIDS.

In addition, it is known that symptpms isn't immediatelyappeared via latent after infection. Anti-retrovirus compositions of the present invention are useful as a preventing agent for the appearance of retrovirus infection of such a latent infection patient.

The present invention has a characteristic in the synergistic effect obtained by combining a compound represented by the formula (I): A-C(=)-CH(OH)-B wherein A and B are as described above, and one or more different type of anti-HIV active substance.

Therefore, each active ingredient can be administered as a single preparation containing them, or administered simultaneously as each separate preparation. In the addition, such a similar synergistic effect can be obtained by successively and separately administrating each active ingredient at a suitable interval not reducing the synergistic effect.

As examples of retrovirus infection which can be treated or prevented by an anti-retrovirus composition of the present invention, included are human immunodeficiency virus infection (e.g., AIDS), human T cell leukemia virus infection (human T cell leukemia, and the like. In the addition, as examples of retrovirus infection against animals, included are feline immunodeficiency virus infection (e.g., feline AIDS), simian immunodeficiency virus infection (e.g., simian AIDS), and the like.

In addition, the anti-retrovirus compositions of the present invention are useful for treating and preventing not only retrovirus infection (e.g., AIDS), but also symptom involved with retrovirus infection (e.g., related clinical symptom such as AIDS-related complex (ARC), and persistent generalized lymphadennopathy (PGL), AIDS-related neuropathic symptom such as multiple sclerosis). Further, as AIDS-related complex involved are Kaposi's sarcoma, Pneumocystis Carinii Pneumonia, HIV encephalopathy, Candida esophagitis, and the like.

In addition, the anti-retrovirus compositions of the present invention can be used for treating asymptomatic infection, incipient infection, or disease caused or supervened by retrovirus.

The anti-retrovirus compositions of the present invention can be administered orally or parenterally. For oral

administration, the anti-retrovirus compositions of the present invention can be used in any form of usual formulations, for example, solid formulations such as tablets, powders, granules, capsules, pills, solutions, syrup, buccals or soblingual formulation. For parenteral administration, the anti-retrovirus compositions of the present invention can be used in any form of usual formulations, for example, injection for intramuscular administration, suppository, percutaneous absorbent, inhalation. Oral administration is preferable.

The anti-retrovirus compositions of the present invention can be prepared by suitably combining an effective amount of active ingredient and various pharmaceutical additives, such as excipient, binder, humectant, disintegrant, lubricant and attenuant, which is suitable to final administration formulation. In the case of injection, the formulation is prepared by sterilization with a suitable carrier.

Concretely, as excipient are included lactose, saccharose, dextrose, starch, calcium carbonate, crystal cellulose, and the like, as binder are included methylcellulose, carboxymethylcellulose, hydroxypropylcellulose, gelatin, polyvinylpyrrolidone, and the like, as disintegrant are included carboxymethylcellulose, sodium carboxymethylcellulose, starch, sodium alginate, agar, sodiumlauryl sodium sulfate, and the like, as lubricant are included talc, magnesium stearate, macrogol, and the like. As base of suppository can be used, cacao adeps, macrogol, methylcellulose, and the like. In addition, when solution or emulsive, suspencible injection is prepared, can be added conveniently to it lysing adjuvant, suspension, emulsifier, stabilizer, preservative, homotonic agent and the like which are usually used, and the like, and in the case of oral administration can be added corrigent, flavor, and the like.

Active ingredient of the anti-retrovirus compositions of the present invention can be composed of the compound represented by the formula (I) and one or more different type of anti-retrovirus active substances.

In single administration of the compound represented by the formula (I) or another anti-retrovirus active substance, the daily dosage of each active ingredient can be between 0.05-3000 mg, preferably 0.1-1000 mg for oral administration, and between 0.01-1000 mg, preferably 0.05-500 mg for parenteral administration. The above mentioned dosage can be determined by measuring CD4 positive cell numbers the amount of virus RNA, e.g. in blood (or blood plasma) after administration. And the dosage may be established by the dependence of patient response against said treatment.

When the anti-retrovirus compositions of the present invention is administrated, it is desirable to establish each amount of the active ingredient s depending on age, body weight, conditions of the patient, and the administration route. The composition can be prepared by suitably combining the anti-retrovirus active substances described above, each dose is approximately 0.1 to 1-fold of that for single administration. Therefore, in administration of the anti-retrovirus compositions of the present invention containing the compound represented by the formula (I) and another anti-retrovirus active substance, the daily dosage of each active ingredient can be between 0.005-3000 mg, preferably 0.01-1000 mg, for oral administration, and between 0.001-1000 mg, preferably 0.005-500 mg for parenteral administration.

Furthermore, when each active substances is administrated simultaneously or continuously as separate formulation, they can be formulated and administrated in similar manners described above.

Examples

Reference examples, such as synthetic examples of the compound represented by the formula (I) and anti-human immunodeficiency virus activity, and examples are described below to explain the present invention in detail, which is not to be construed as limiting the scope thereof.

A compound represented by the formula (I) is synthesized by the method described in PCT/JP99/07101.

Synthetic example 1 : 1-[5-(4-fluorobenzyl)furan-2-yl]-3-hydroxy-3-(1*H*-1,2,4-triazol-3-yl)propenone, compound (I-16).

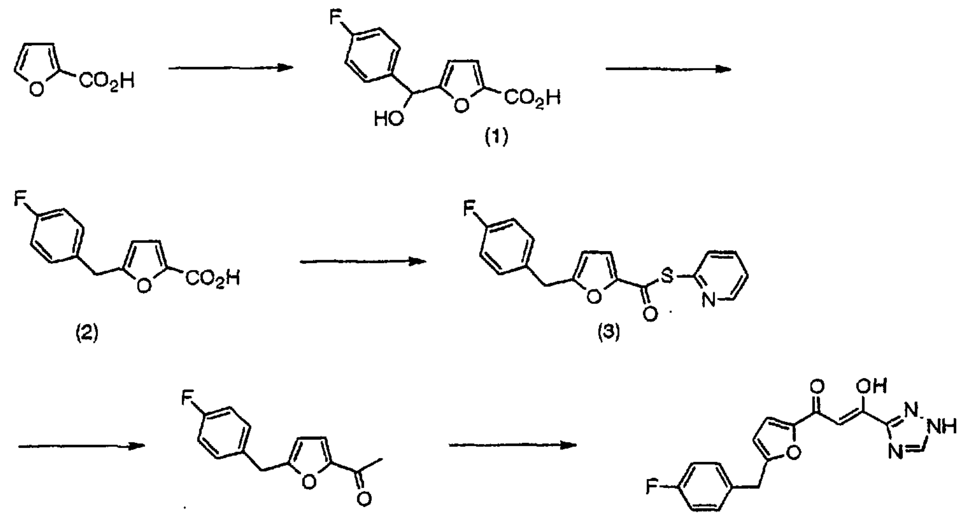

(1)

(2)

(3)

(4)

(5) compound (I-16)

(1) 2-Furancarboxylic acid (5.6 g, 50 mmol) was reacted with 4-fluorobenzaldehyde (6.8 g, 55 mmol) according to the method described in Tetrahedron Letters, 1979, 5, p.469. After post-treatment, the obtaining crude crystal was washed with isopropyl ether to obtain 5-[[1-(4-fuluorophenyl)-1-hydroxy]methyl]furan-2-carboxylic acid (8.1 g, yield 69%).
m.p. : 139-140 °C.
NMR ($CDCl_3$) δ: 5.88(1H, s), 6.28(1H, d, J=3.6Hz), 7.07(2H, t, J=8.7Hz), 7.26(1H, d, J=3.6Hz), 7.39-7.44(2H, m).
(2) The above-mentioned compound (4.72 g, 20 mmol) was reduced with trimethylchlorosilane (10.8 g, 100 mmol) and sodium iodide (15 g, 100 mmol) according to the method described in Tetrahedron 1995, 51, p.11043 to obtain 5-(4-fluorobenzyl)-furan-2-carboxylic acid (3.52 g, yield 80%) as a crystal.
NMR ($d_6$-DMSO) δ: 4.05(2H, s), 6.31(1H, d, J=3.3Hz), 7.12-7.18(3H, m), 7.27-7.32(2H, m), 12.9(1H, brs).
(3) The above-mentioned compound (3.52 g, 16 mmol) was reacted with dipyridyldislufide (4.2 g, 19.2 mmol) and triphenylphosphine (5.04 g, 19.2 mmol) according to the method described in Bull. Chem. Soc. Japan., 1974, 47, p.1777 to obtain 5-(4-fluorobenzyl)furan-2-carboxylic acid 2-pyridylthio ester (3.7 g, yield 77%).
m.p.: 88-89 °C.
NMR ($CDCl_3$) δ: 4.04(2H, s), 6.15(1H, d, J=3.3Hz), 7.03(2H, t, J=8.7Hz), 7.22(1H, d, J=3.3Hz), 7.22-7.26(2H, m), 7.29-7.34(1H, m), 7.70-7.79(2H, m), 8.63-8.66(1H, m).
(4) The above-mentioned compound (3.7 g, 12.4 mmol) was reacted with methyl magnesium bromide THF solution (1M, 14 ml) according to the method described in Bull. Chem. Soc. Japan. 1974, 47, p.1777 to obtain 2-acetyl-5-(4-fluorobenzyl)furan (2.7 g) as an oil.
NMR ($CDCl_3$) δ: 2.43(3H, s), 4.01(2H, s), 6.10(1H, d, J=3.6Hz), 7.01(2H, t, J=9.0Hz), 7.10(1H, d, J=3.6Hz), 7.18-7.23(2H, m).
(5) A solution of the above-mentioned compound (1.31 g, 6 mmol) in THF (18 mL) was cooled and to the reaction mixture was added dropwise a 1 M solution of lithium bistrimethylsilylamide in THF (7.8 mL, 7.8 mmol) keeping at -70-65 °C. Next the reaction mixture was gradually wormed to -10 °C and cooled again to -70 °C, a solution of 1-trityl-1*H*-1,2,4-triazole-3-carboxylic acid ethyl ester (2.99 g, 7.8 mmol) in THF (30 mL) was added dropwise to the reaction mixture. The reaction mixture was gradually wormed to room temperature, and stirred for 1.5 h. The reaction mixture was added to a aqueous solution of excess ammonium chloride, extracted with ethyl acetate, washed with brine, and dried. The solvent was removed and to the residue were added dioxane (75 mL) and 1mol/$dm^3$ HCl (20 mL), the reaction mixture was heated with stirring at 80 °C for 0.5 h. Next dioxane was removed under reduced pressure and the residue was partitioned between ethyl acetate and water. The ethyl acetate layer was washed with water and dried. The solvent was removed and the residue was dissolved in ether. The ether solution was extracted with 1 mol/$dm^3$ NaOH (6 mL) for three times. The alkaline extract was washed with ether for two times, neutralized with 1 mol/$dm^3$ HCl, and extracted with ethyl acetate. The extract was washed with water, brine, and dried. The solvent was removed and the obtaining crude crystal was washed with small amount of ethyl acetate,

recrystallized from ethyl acetate to obtain a title compound (1.15 g, yield 61%).
m.p. : 183-185 °C.
Analytical Calcd. for $C_{16}H_{12}FN_3O_3$: C, 61.34; H, 3.86; N, 13.41; F, 6.06. Found: C, 61.22; H, 3.72; N, 13.41; F, 6.03.
NMR ($d_6$-DMSO) δ: 4.15(2H, s), 6.47(1H, d, J=3.3Hz), 6.93(1H, s), 7.17(2H, t, J=9.0Hz), 7.31-7.37(2H, m), 7.50 (1H, d, J=3.3Hz), 8.70(1H, brs).

Synthetic examples 2-51

Compound (I-1) to (I-15), (I-17) to (I-51) were synthesized in a similar manner as above. Physical data of each compound were described below.

Compound (I-1)

m.p. : 203-206 °C (solvent for recrystallization : ethyl acetate).
Analytical Calcd. for $C_{15}H_{12}FN_5O_2$: C, 57.51; H, 3.86; N, 22.35; F, 6.06. Found: C, 57.10; H, 3.89; N, 22.23; F, 5.79.
NMR ($d_6$-DMSO) δ: 5.39(2H, s), 6.92(1H, s), 7.17-7.41(4H, m), 8.14(1H, s), 8.66(1H, brs), 8.76(1H, s), 14.3(1H, brs).

Compound (I-2)

m.p. : 187-191 °C (solvent for recrystallization : ethyl acetate).
Analytical Calcd. for $C_{16}H_{12}FN_3O_3$: C, 61.34; H, 3.86; N, 13.41; F, 6.06. Found: C, 61.08; H, 3.87; N, 13.72; F, 6.08.
NMR ($d_6$-DMSO) δ: 3.81(2H, s), 6.97(1H, s), 7.14(2H, t, J=9.0Hz) 7.30-7.35(2H, m), 7.45(1H, s), 7.92(1H, s), 8.75(1H, brs).

Compound (I-3)

m.p. : 121-123 °C (solvent for recrystallization : ether).
Analytical Calcd. for $C_{15}H_{11}FN_4O_3$: C, 57.33; H, 3.53; N, 17.83; F, 6.04. Found: C, 57.25; H, 3.58; N, 17.53; F, 5.81.
NMR ($d_6$-DMSO) δ: 4.16(2H, s), 6.51(1H, d, J=3.6Hz), 7.05(1H, s), 7.18(2H, t, J=8.7Hz), 7.32.7.38(2H, m), 7.65(1H, d, J=3.6Hz).

Compound (I-4)

m.p. : 144-147 °C (solvent for recrystallization : ethyl acetate).
Analytical Calcd. for $C_{15}H_{11}N_3O_3S$ 0.3 $H_2O$: C, 56.52; H, 3.67; N, 13.18; S, 10.06. Found: C, 56.85; H, 3.71; N, 13.56; S, 9.48.
NMR ($d_6$-DMSO) δ: 6.97(1H, s), 7.12(1H, d, J=3.6Hz), 7.30-7.44(5H, m), 7.65(1H, d, J=3.6Hz), 8.74(1H, brs).

Compound (I-5)

m.p. : 207-210 °C (solvent for recrystallization : ethyl acetate).
Analytical Calcd. for $C_{15}H_{11}N_3O_5S$ 1.2 $H_2O$: C, 49.10; H, 3.68; N, 11.45; S, 8.74. Found: C, 48.84; H, 3.68; N, 11.67; S, 9.05.
NMR($d_6$-DMSO) δ: 7.02(1H, s), 7.62-7.86(5H, m), 8.02-8.08(2H, m), 8.82(1H, brs).

Compound (I-6)

m.p. : 72-73 °C (solvent for recrystallization : ether).
Analytical Calcd. for $C_{13}H_{15}N_3O_3$ 0.25 $H_2O$: C, 58.75; H, 5.88; N, 15.81. Found: C, 58.10; H, 5.65; N, 15.81.
NMR ($CDCl_3$) δ: 0.96(3H, t, J=7.5Hz), 1.35-1.42(2H, m), 1.65-1.75(2H, m), 2.74(2H, t, J=7.5Hz), 6.25(1H, d, J=3.6Hz), 7.12(1H, s), 7.29(1H, d, J=3.6Hz).8.44(1H, s).

Compound (I-7)

m.p. : 185-187 °C (solvent for recrystallization : ethyl acetate).

Analytical Calcd. for $C_{16}H_{12}FN_3O_2S$ 0.3 $H_2O$: C, 57.41; H, 3.79; N, 12.55; F, 5.68; S, 9.58. Found: C, 57.58; H, 3.82; N, 12.77; F, 5.49; S, 9.31.
NMR ($d_6$-DMSO) δ: 4.25(2H, s), 7.04-7.40(6H, m), 7.98(1H, d, J=3.8Hz), 8.77(1H, brs), 13.8(1H, brs).

Compound (I-8)

m.p. : 124-125 °C (solvent for recrystallization : ether-hexane).
Analytical Calcd. for $C_{12}H_{14}N_4O_3$: C, 54.96; H, 5.38; N, 21.36. Found: C, 55.02; H, 5.43; N, 21.09.
NMR ($CDCl_3$) δ: 0.95(3H, t, J=7.8Hz), 1.37-1.45(2H, m), 1.65-1.73(2H, m), 2.76(2H, t, J=7.8Hz), 6.30(1H, d, J=3.6Hz), 7.23(1H, s), 7.39(1H, d, J=3.6Hz).

Compound (I-9)

m.p. : 176-179 °C (solvent for recrystallization : ethyl acetate).
Analytical Calcd. for $C_{16}H_{12}FN_3O_3$: C, 61.34; H, 3.86; N, 13.41; F, 6.06. Found: C, 61.19; H, 3.81; N, 13.52; F, 6.19.
NMR ($d_6$-DMSO) δ: 4.03(2H, s), 6.62(1H, d, J=0.9Hz), 6.90(1H, s), 7.15(2H, t, J=9.0Hz), 7.29-7.34(2H, m), 8.60(1H, d, J=0.9Hz), 8.67(1H, brs).

Compound (I-10)

m.p. : 180-183 °C.
Analytical Calcd. for $C_{16}H_{13}FN_4O_2$ 0.2 $H_2O$: C, 60.83; H, 4.28; N, 17.74; F, 6.01. Found: C, 60.61; H, 4.24; N, 17.61; F, 5.82.
NMR($d_6$-DMSO) δ: 3.97(2H, s), 6.04(1H,s) 6.89(1H, s), 7.01-7.18(6H, m), 8.72(1H, brs).

Compound (I-11)

m.p. : 171-174 °C (solvent for recrystallization : ether).
Analytical Calcd. for $C_{15}H_{11}FN_4O_3$: C, 57.33; H, 3.53; N, 17.86; F, 6.05. Found: C, 57.05; H, 3.61; N, 17.74; F, 5.82.
NMR ($d_6$-DMSO) δ: 4.24(2H, s), 6.70(1H, d, J=1.8Hz), 7.09(1H, s), 7.10-7.17(2H, m), 7.32-7.37(2H, m), 8.04(1H, d, J=1.8Hz).

Compound (I-12)

m.p. : 221-223 °C (solvent for recrystallization : ether).
Analytical Calcd. for $C_{16}H_{12}FN_3O_3$: C, 61.34; H, 3.86; N, 13.41; F, 6.06. Found: C, 61.04; H, 3.98; N, 13.28; F, 5.87.
NMR ($d_6$-DMSO) δ: 4.23(2H, s), 6.65(1H, d, J=1.8Hz), 7.03(1H, s), 7.10-7.16(2H, m), 7.31-7.36(2H, m), 7.97(1H, d, J=1.8Hz), 8.74(1H, brs), 14.7(1H, brs).

Compound (I-13)

m.p. : 217-220 °C (solvent for recrystallization : ethyl acetate).
Analytical Calcd. for $C_{16}H_{12}FN_3O_3$: C, 61.34; H, 3.86; N, 13.41; F, 6.06. Found: C, 61.19; H, 4.04; N, 13.16; F, 5.90.
NMR($d_6$-DMSO) δ: 4.02(2H, s), 6.93(1H, s), 7.09(2H, t, J=9.0Hz), 7.25-7.31(2H, m), 7.56(1H, s), 8.66(1H, brs), 8.80 (1H, s).

Compound (I-14)

m.p.: 195-197 °C (solvent for recrystallization : ether).
Analytical Calcd. for $C_{14}H_{11}FN_6O_2$: C, 53.50; H, 3.53; N, 26.74; F, 6.04. Found: C, 53.65; H, 3.53; N, 26.71; F, 5.92.
NMR ($d_6$-DMSO) δ: 5.79(2H, s), 7.12-7.26(5H, m), 7.47(1H, d, J=2.1H 7.74(1H, d, J=2.1Hz).

Compound (I-15)

m.p. : 189-191 °C.

Analytical Calcd. for $C_{18}H_{17}FN_4O_3$ 0.2 $C_4H_8O_2$: C, 60.38; H, 5.01; N, 14.98; F, 5.04. Found: C, 60.53; H, 4.79; N, 14.71; F, 5.05.

NMR ($d_6$-DMSO) δ: 3.20(3H, s), 5.20(2H, s), 6.68(1H, s), 6.88(1H, s), 7.02-7.25(2H, m), 7.32-7.42(2H, m), 8.05(1H, brs), 8.75(1H, brs).

Compound (I-17)

m.p. : 210-211 °C (solvent for recrystallization : chloroform).

Analytical Calcd. for $C_{19}H_{19}FN_4O_2$: C, 64.40; H, 5.40; N, 15.81; F, 5.36. Found: C, 64.28; H, 5.37; N, 15.61; F, 5.23.

NMR ($d_6$-DMSO) δ: 0.81(3H, t, J=7.2Hz), 1.60-1.82(2H, m), 3.68-3.96(2H, m), 4.05(2H, s), 6.57(1H, d, J=1.8Hz), 6.80-7.36(4H, m), 7,91(1H, d, J=1.8Hz), 8.57(1H, brs).

Compound (I-18)

m.p. ; 166-168 °C (solvent for recrystallization : ethyl acetate).

Analytical Calcd. for $C_{23}H_{18}F_2N_4O_2$: C, 65.71; H, 4.32; N, 13.33; F, 9.04. Found: C, 65.82; H, 4.33; N, 13.03; F, 8.78.

NMR ($d_6$-DMSO) δ: 4.03(2H, s), 5.10(2H, s), 6.65(1H, s), 6.84(1H, s), 7.00-7.40(8H, m), 8.04(1H, s), 8.58(1H, brs).

Compound (I-19)

m.p. : 210-213 °C (solvent for recrystallization : ethyl acetate-hexane)

Analytical Calcd. for $C_{16}H_{13}FN_4O_2$ 0.2 $H_2O$, 0.2 $C_2H_6O$: C, 60.59; H, 4.53; N, 17.23; F, 5.84. Found: C, 60.62; H, 4.45; N, 16.95; F, 5.69.

NMR ($d_6$-DMSO) δ: 4.07(2H, s), 6.59(1H, s), 6.87(1H, s), 7.02-7.11(2H, m), 7.16-7.30(2H, m), 7.83(1H, s), 8.70(1H, brs), 11.5(1H, s), 14.6(1H, brs).

Compound (I-20)

m.p. : 150-153 °C (solvent for recrystallization : ether).

Analytical Calcd. for $C_{15}H_{11}FN_4O_3$: C, 57.33; H, 3.53; N, 17.83; F, 6.04. Found: C, 57.29; H, 3.72; N, 17.74; F, 5.84.

NMR ($CDCl_3$) δ: 4.40(2H, s), 6.75(1H, d, J=2.1Hz), 6.99(2H, t, J=8.7Hz), 7.01(1H, s), 7.24-7.29(2H, m), 7.39(1H, d, J=2.1Hz).

Compound (I-21)

m.p. : 178-181 °C (solvent for recrystallization : ether).

Analytical Calcd. for $C_{22}H_{19}N_5O_4S$: C, 58.79; H, 4.26; N, 15.58; S, 7.13. Found: C, 59.24; H, 4.37; N, 15.75; S, 6.61.

NMR ($d_6$-DMSO) δ: 2.81-2.98(4H, m), 7.11-7.30(7H, m), 7.68-7.73(2H, m), 7.80-7.86(1H, m), 8.07-7.10(2H, m), 8.64 (1H, d, J=2.4Hz).

Compound (I-22)

m.p. : 228-230 °C (solvent for recrystallization : ether)

Analytical Calcd for $C_{16}H_{15}N_5O_2$ 0.16 $C_2H_4O_2$: C, 61.46; H, 4.94; N, 21.96. Found: C, 61.74; H, 4.88; N, 21.67.

NMR ($d_6$-DMSO) δ: 2.84-3.02(4H, m), 6.74(1H, m), 7.04(1H, s), 7.17-7.32(5H, m), 7.96(1H, m), 11.6(1H, brs).

Compound (I-23)

m.p. : 215-218 °C (solvent for recrystallization : chloroform).

Analytical Calcd. for $C_{19}H_{16}N_4O_4$: C, 62.63; H, 4.43; N, 15.38. Found: C, 62.29; H, 4.69; N, 15.11.

NMR ($d_6$-DMSO) δ: 5.19(2H, s), 6.25(1H, d, J=16.5Hz), 6.90(1H, s), 7.30-7.43(5H, m), 7.72(1H, s), 8.12-8.22(2H, m),

8.62(1H, brs).

Compound (I-24)

m.p. : 226-228 °C (solvent for recrystallization : ethyl acetate-hexane).
Analytical Calcd. for $C_{19}H_{16}N_4O_4$ 0.1 $C_4H_8O_2$: C, 62.44; H, 4.54; N, 15.01. Found: C, 62.06; H, 4.61; N, 14.82. NMR ($d_6$-DMSO) δ: 5.19(2H, s), 6.25(1H, d, J=16.2Hz), 6.90(1H, s), 7.28-7.44(5H, m), 7.72(1H, s), 8.12-8.20(2H, m), 8.63(1H, brs).

Compound (I-25)

m.p. : 144-147 °C (solvent for recrystallization : ethyl acetate)
Analytical Calcd. for $C_{16}H_{12}FN_3O_3$ 0.1 $H_2O$: C, 60.99; H, 3.90; N, 13.34; F, 6.03. Found: C, 61.01; H, 4.07; N, 13.47; F, 5.99.
NMR ($d_6$-DMSO) δ: 4.41(2H, s), 6.92(1H, s), 7.04(1H, d, J=1.8Hz), 7.14(2H, t, J=9.3Hz), 7.28-7.33(2H, m), 7.72(1H, d, J=1.8Hz), 8.70(1H, brs).

Compound (I-26)

m.p. : 168-170 °C.
Analytical Calcd. for $C_{15}H_{12}FN_5O_2$ 0.6 $H_2O$: C, 53.75; H, 3.79; N, 20.90; F, 5.67. Found: C, 53.83; H, 3.73; N, 21.20; F, 5.50.
NMR ($d_6$-DMSO) δ: 5.20(2H, s), 6.68(1H, dd, J=3.0 , 1.8Hz), 6.96(1H, s), 7.02-7.08(1H, s), 7.16-7.26(2H, m), 7.34-7.44 (2H, m), 8.02-8.08(1H, m).

Compound (I-27)

m.p. : 190-195 °C (solvent for recrystallization : ether).
Analytical Calcd. for $C_{20}H_{14}FN_3O_2S$: C, 63.31; H, 3.72; N, 11.08; F, 5.01; S, 8.45. Found: C, 63.08; H, 3.82; N, 11.28; F, 4.84; S, 8.46.
NMR ($CDCl_3$) δ: 4.53(2H, s), 7.01(2H, t, J=8.7Hz), 7.11(1H, s), 7.26-7.48(4H, m), 7.75(1H, d, J=7.8Hz), 8.10(1H, d, J=7.5Hz), 8.43(1H, brs).

Compound (I-4)

m.p. : 122-124 °C (solvent for recrystallization : ether-hexane).
Analytical Calcd. for $C_{20}H_{14}FN_3O_3$ 0.25 $H_2O$: C, 65.30; H, 3.97; N, 11.42; F, 5.16. Found: C, 65.50; H, 3.99; N, 11.24; F, 4.99.
NMR ($CDCl_3$) δ: 4.54(2H, s), 6.98-7.04(2H, m), 7.26(1H, s), 7.34-7.41(4H, m), 7.47-7.50(1H, m), 7.96-7.99(1H, m), 8.38(1H, s).

Compound (I-29)

m.p. : 264-265 °C.
Analytical Calcd. for $C_{17}H_{14}N_4O_4$ 0.3 $H_2O$: C, 59.40; H, 4.28; N, 16.30. Found: C, 59.21; H, 4.30; N, 16.20. NMR ($d_6$-DMSO) 6: 5.63(2H, s), 6.95(1H, s), 7.16.7.40(6H, m), 8.27(1H, d, J=1.8Hz), 8.69(1H, brs), 12.8(1H, brs).

Compound (I-30)

m.p. : 204-206 °C.
Analytical Calcd. for $C_{19}H_{18}N_4O_4$: C, 62.29; H, 4.95; N, 15.29. Found: C, 61.94; H, 5.03; N, 15.02.
NMR ($d_6$-DMSO) δ: 1.24(3H, t, J=7.2Hz), 4.20(2H, q, J=7.2Hz), 5.61(2H, s), 6.97(1H, s), 7.12-7.42(6H, m), 8.32(1H, s), 8.77(1H, s).

Compound (I-31)

m.p. : 238-240 °C.
Analytical Calcd. for $C_{20}H_{18}N_4O_4$ 0.4$H_2O$: C, 62.30; H, 4.91; N, 14.53. Found: C, 62.18; H, 4.74; N, 14.53.

NMR ($d_6$-DMSO) δ: 3.64(3H, s), 5.42(2H, s), 6.36(1H, d, J=15.9Hz), 6.95(1H, s), 7.02-7.58(7H, m), 8.17(1H, s), 8.76 (1H, s).

Compound (I-32)

m.p. : 170-173 °C (solvent for recrystallization : ethyl acetate).
Analytical Calcd. for $C_{16}H_{13}FN_4O_2$ 0.2 HCl: C, 60.13; H, 4.16; N, 17.53; F, 5.94. Found: C, 60.21; H, 4.12; N, 17.41; F, 5.62.
NMR ($d_6$-DMSO) δ: 5.65(2H, s), 6.30-6.36(1H, m), 6.94(1H, m), 7.04-7.50(6H, m), 8.65(1H, brs).

Compound (I-33)

m.p.: 147-150 °C.
Analytical Calcd. for $C_{15}H_{12}FN_5O_2$ 0.2 $H_2O$: C, 56.85; H, 3.94; N, 22.10; F, 6.00. Found: C, 56.68; H, 4.02; N, 22.47; F, 5.72.
NMR ($d_6$-DMSO) δ: 5.65(2H, s), 6.37(1H, dd, J=3.0, 2.7Hz), 7.05-7.20(5H, m), 7.47-7.58(2H, m).

Compound (I-34)

m.p. : 258-264 °C (solvent for recrystallization : chloroform).
Analytical Calcd. for $C_{15}H_{12}N_4O_4S$: C, 52.32; H, 3.51; N, 16.27; S, 9.31. Found: C, 52.26; H, 3.60; N, 16.05; S, 9.22.
NMR ($d_6$-DMSO) δ: 6.85(1H, m), 7.03(1H, s), 7.53(1H, m), 7.67-7.74(2H, m), 7.78-7.84(1H, m), 8.08-8.15(2H, m), 8.37 (1H, m), 8.65(1H, brs).

Compound (I-35)

m.p. : 164-168 °C (solvent for recrystallization : ether-hexane)
Analytical Calcd. for $C_{19}H_{13}FN_4O_3$ 0.2 $C_4H_{10}O$: C, 62.72; H, 3.99; N, 14.78; F, 5.01. Found: C, 62.43; H, 3.74; N, 14.74; F, 4.76.
NMR($CDCl_3$) δ: 4.53(2H, s), 6.98-7.04(2H, m), 7.26(1H, s), 7.34-7.41(4H, m), 7.49-7.52(1H, m), 7.95-7.98(1H, m).

Compound (I-36)

m.p. : 181-183 °C (solvent for recrystallization : chloroform).
Analytical Calcd. for $C_{19}H_{14}N_4O_3$ 0.25 $H_2O$: C, 65.04; H, 4.17; N, 15.97. Found: C, 65.02; H, 3.96; N, 16.10.
NMR ($d_6$-DMSO) δ: 4.61(2H, s), 7.17(1H, s), 7.26-7.47(7H, m), 7.68-7.70(1H, m), 7.95-7.98(1H, m).

Compound (I-37)

m.p. : 178-180 °C (solvent for recrystallization : ethyl acetate).
NMR ($d_6$-DMSO) δ: 1.19(3H, t, J=7.6Hz), 2.73(2H, qd, J=7.6 , 1.2Hz), 6.96-7.04(2H, m), 7.48-7.78(3H, m), 7.90-8.02(3H, m).

Compound (I-38)

m.p. : 216-217 °C.
Analytical Calcd. for $C_{16}H_{14}N_4O_2$ 0.05 $CHCl_3$ 0.2 $H_2O$: C, 63.44; H, 4.79; N, 18.44. Found: C, 63.47; H, 4.79; N, 18.39.
NMR ($d_6$-DMSO) δ: 5.20(2H, s), 6.60-6.62(1H, m), 6.84(1H, s), 7.00-7.02(1H, m), 7.29-7.40(5H, m), 7.92-7.93(1H, m), 8.69(1H, brs), 14.6(1H, brs).

Compound (I-39)

m.p. : 178-180 °C (solvent for recrystallization : ethyl acetate-hexane-ether).
Analytical Calcd. for $C_{19}H_{19}N_5O_4$: C, 59.84; H, 5.02; N, 18.36. Found: C, 59.38; H, 5.07; N, 18.03.
NMR ($CDCl_3$) δ: 2.50-2.70(2H, m), 2.75-2.88(2H, m), 3.72(3H, s), 5.10(2H, s), 6.48(1H, s), 6.95(1H, s), 7.02-7.10(2H, m), 7.28-7.48(4H, s).

Compound (I-40)

m.p. : 223-225 °C (solvent for recrystallization : ethyl acetate-hexane).

Analytical Calcd. for $C_{19}H_{17}N_5O_4$ 0.1 $C_4H_8O_2$: C, 60.03; H, 4.62; N, 18.04. Found: C, 60.26; H, 4.57; N, 17.91. NMR ($d_6$-DMSO) δ: 3.66(3H, s), 5.48(2H, s), 6.49(1H, d, J=15.6Hz), 7.08(1H, s), 7.10-7.60(7H, m), 8.30(1H, s).
Compound (I-41)
m.p. : 85-90 °C (solvent for recrystallization : ethyl acetate)
NMR ($d_6$-DMSO) δ: 1.36(3H, t, J=6.9Hz), 4.30(2H, q, J=6.9Hz), 5.62(2H, s), 7.04(1H, s), 7.16-7.62(5H, m).

Compound (I-42)

m.p. : 118-120 °C (solvent for recrystallization : ether-hexane-ethyl acetate)

Analytical Calcd. for $C_{19}H_{21}N_5O_2$ 0.2 $C_4H_8O_2$: C, 64.44; H, 6.17; N, 18.98. Found: C, 64.60; H, 6.02; N, 18.97. NMR ($CDCl_3$) δ: 0.91(3H, t, J=7.2Hz), 1.30-1.44(2H, m), 1.52-1.68(2H, m), 2.46(2H, t, J=7.2Hz), 5.09(2H, s), 6.50(1H, s), 7.04-7.10(2H, m), 7.30-7.50(3H, m).

Compound (I-43)

m.p. : 156-158 °C (solvent for recrystallization : ethyl acetate-hexane).

Analytical Calcd. for $C_{18}H_{19}N_5O_2$ 0.1 $C_4H_8O_2$: C, 63.84; H, 5.77; N, 20.23. Found: C, 63.93; H, 5.76; N, 20.23. NMR ($d_6$-DMSO) δ: 0.89(3H, t, J=7.8Hz), 1.42-1.60(2H, m), 2.42(2H, t, J=7.8Hz), 5.23(2H, s), 6.47(1H, s), 6.95(1H, s), 7.10-7.18(2H, m), 7.26-7.40(3H, m), 7.99(1H, s).

Compound (I-44)

m.p. : 188-189 °C (solvent for recrystallization : ethyl acetate).

Analytical Calcd. for $C_{15}H_{13}N_5O_2$ 0.2 $H_2O$: C, 60.27; H, 4.52; N, 23.43. Found: C, 60.39; H, 4.51; N, 23.39. NMR ($d_6$-DMSO) δ: 5.22(2H, s), 6.68-6.69(1H, m), 6.97(1H, s), 7.05(1H, m), 7.31-7.40(5H, m), 8.06(1H, m).

Compound (I-45)

m.p. : 203-204 °C (solvent for recrystallization : ethyl acetate).

Analytical Calcd. for $C_{14}H_{11}N_5O_4$ S 0.75 $H_2O$: C, 46.86; H, 3.51; N, 19.52; F, 8.94. Found: C, 47.22; H, 3.48; N, 19.32; F, 8.95.
NMR ($d_6$-DMSO) δ: 6.89-6.92(1H, m), 7.21(1H, s), 7.56-7.57(1H, m), 7.68-7.73(2H, m), 7.80-7.84(1H, m), 8.12-8.15 (2H, m), 8.52-8.53(1H, m).

Compound (I-46)

m.p. : 84-85 °C (solvent for recrystallization : ether-hexane).

Analytical Calcd. for $C_{19}H_{14}FNO_3$ 0.2$H_2O$: C, 69.80; H, 4.44; N, 4.28; F, 5.81. Found: C, 69.76; H, 4.34; N, 4.34; F, 5.73.
NMR ($CDCl_3$) δ: 4.06(2H, s), 6.16(1H, d, J=3.3Hz), 7.03(2H, t, J=8.4Hz), 7.20-7.30(3H, m), 7.32(1H, s), 7.40-7.48(1H, m), 7.87(1H, dt, J=1.5, 7.5Hz), 8.11(1H, d, J=7.5Hz), 8.68-8.74(1H, m).

Compound (1-47)

m.p. : 77-80 °C (solvent for recrystallization : ethyl acetate-chloroform).

Analytical Calcd. for $C_{18}H_{13}FNO_3$ 0.2$H_2O$ 0.2$C_4H_8O_2$ 0.03$CHCl_3$: C, 64.78; H, 4.34; N, 8.02; F, 5.44. Found: C, 65.04; H, 4.04; N, 7.77; F, 5.56.
NMR ($CDCl_3$) δ: 4.07(2H, s), 6.18(1H, d, J=3.2Hz), 7.03(2H, t, J=8.8Hz), 7.18-7.22(3H, m), 7.39(1H, s), 7.39(1H, t, J=4.8Hz), 8.92(2H, d, J=4.8Hz).

Compound (I-48)

m.p. : 196-198 °C (solvent for recrystallization : isopropyl ether).

Analytical Calcd. for $C_{20}H_{14}FNO_5$ 0.2$H_2O$: C, 64.76; H, 3.91; N, 3.78; F, 5.12. Found: C, 64.95; H, 3.73; N, 3.93; F, 4.99.

NMR ($CDCl_3$) δ: 4.08(2H, s), 6.18(1H, d, J=3.6Hz), 7.03(2H, t, J=9.0Hz), 7.20-7.32(3H, m), 7.37(1H, s), 8.20(1H, d, J=8.4Hz), 8.51(1H, dd, J=8.4, 1.8Hz), 9.34(1H, brs).

Compound (I-49)

m.p. : 208-210 °C (solvent for recrystallization : isopropyl ether).

Analytical Calcd. for $C_{20}H_{14}FNO_5$: C, 65.40; H, 3.84; N, 3.81; F, 5.17. Found: C, 65.14; H, 3.79; N, 3.90; F, 4.95. NMR ($CDCl_3$) δ: 4.09(2H, s), 6.25(1H, d, J=3.6Hz), 7.03(2H, t, J=8.4Hz), 7.21-7.32(3H, m), 7.65(1H, s), 7.96-8.02(1H, m), 8.56(1H, brs), 8.85(1H, d, J=5.1Hz).

Compound (I-50)

m.p. : 108-109 °C (solvent for recrystallization : ethyl acetate-isopropyl ether).

Analytical Calcd. for $C_{18}H_{13}FN_2O_3$: C, 66.66; H, 4.04; N, 8.64; F, 5.86. Found: C, 66.64; H, 3.96; N, 8.66; F, 5.59.
NMR ($CDCl_3$) δ: 4.19(2H, s), 7.02-7.07(2H, m), 7.26-7.34(3H, m), 7.45-7.48(1H, m), 7.80(1H, s), 7.86-7.91(1H, m), 8.12(1H, d, J=7.8Hz), 8.72(1H, d, J=4.5Hz).

Compound (I-51)

m.p. : 97-100 °C (solvent for recrystallization : ethyl acetate-isopropyl ether).

Analytical Calcd. for $C_{17}H_{12}FN_3O_3$ $0.4H_2O$: C, 61.41; H, 3.88; N, 12.64; F, 5.71. Found: C, 61.76; H, 3.58; N, 12.21; F, 5.84.
NMR ($d_6$-DMSO) δ: 4.28(2H, s), 7.15-7.21(3H, m), 7.36-7.40(2H, m), 7.64(1H, brs), 8.11(1H, brs), 8.98-9.02(2H, m).

Reference example 1

Inhibitory activities of a compound represented by the formula of A-C(=O)-CH=C(OH)-B against integrase of each retrovirus is described.

The inhibitory effects of the compounds of the present invention for HIV-1 integrase have been determined by the assay described below.

(1) Preparation of DNA solutions.

Substrate DNA and target DNA, which sequences were indicated below, were synthesized by Amersham Pharmacia Biotech and dissolved in KTE buffer (composition: 100 mM KCl, 1 mM EDTA, 10 mM Tris-HCl (pH 7.6)) at concentration of 2 pmol/μL and 5 pmol/μL, respectively. The DNA solutions were annealed with each complement by slowly cooling after heating.

(Substrate DNA)

5'- Biotin-ACC CTT TTA GTC AGT GTG GAA AAT CTC TAG CAG T-3'

3'- GAA AAT CAG TCA CAC CTT TTA GAG ATC GTC A-5'

(Target DNA)

5'- TGA CCA AGG GCT AAT TCA CT-Dig-3'

3'-Dig-ACT GGT TCC CGA TTA AGT GA -5'

(2) Calculations of the percent inhibitions (the $IC_{50}$ values of test compounds)

Streptavidin, obtained from Vector Laboratories, was dissolved in 0.1 M carbonate buffer (composition: 90 mM $Na_2CO_3$, 10 mM $NaHCO_3$) at concentration of 40 μg/mL. After coating each well of microtiter plates (obtained from NUNC) with 50 μL of the above solution at 4 °C over night, each well was washed twice with PBS (composition: 13.7 mM NaCl 0.27 mM KCl, 0.43 mM $Na_2HPO_4$, 0.14 mM $KH_2PO_4$) and blocked with 300 μL of 1% skim milk in PBS for 30 min. Additionally, each well was washed twice with PBS and added 50 μL of substrate DNA solution (2 pmol/μL). The microtiter plates were kept at room temperature for 30 min. Then, each well was washed twice with PBS and once with $H_2O$.

Subsequently, in the each well prepared above were added 45 μL of the reaction buffer prepared from 12 μL of the buffer (composition: 150 mM MOPS (pH 7.2), 75 mM $MnCl_2$, 50 mM 2-mercaptoethanol, 25% glycerol, 500 μg/mL bovine serum albumin-fraction V), 1 μL of target DNA (5 pmol/μL), and 32 μL of the distilled water. Additionally, 6 μL of either a test compound in DMSO or DMSO for positive control(PC) was mixed with the above reaction buffer, then 9 μL of an integrase solution (30 pmol) was added and mixed well. In the well of negative control (NC) was added 9 μL of the integrase dilution buffer (composition: 20 mM MOPS (pH7.2), 400 mM potassium glutamate, 1 mM EDTA, 0.1% NP-40, 20% glycerol, 1 mM DTT, 4M urea).

The microtiter plates were incubated at 30 °C for 1 hour. The reaction solution was removed and each well was washed twice with PBS. Subsequently, each well of the microtiter plates was filled with 100 μL of anti-digoxigenin antibody labeled with alkaline phosphatase (Sheep Fab fragment: obtained from Boehringer) and incubated at 30 °C for 1 hour. Then, each well was washed twice with 0.05% Tween20 in PBS and once with PBS. Next, 150 μL of the Alkaline phosphatase reaction buffer (composition: 10mM *p*-Nitrophenylphosphate (obtained from Vector Laboratories), 5 mM $MgCl_2$, 100 mM NaCl 100 mM Tris-HCl (pH 9.5))was added in each well. The microtiter plates were incubated at 30 °C for 2 hours and the reaction was terminated by the addition of 50 μl of 1 N NaOH solution. The optical density (OD) at 405 nm of each well was measured and the percent inhibition was determined by the following expression.

The percent inhibition (%) = 100[1-{(C abs.- NC abs.) / (PC abs.- NC abs.)}]

C abs. ; the OD of the well of the compounds
NC abs. : the OD of the negative control (NC)
PC abs. : the OD of the positive control (PC)

When the percent inhibition (%) is X% at the concentration of x μg/mL and the percent inhibition (%) is Y% at the concentration of y μg/mL, one of which is more than 50% and the other is less than 50%, $IC_{50}$ can be determined by the following expression.

$$IC_{50}(\mu g/mL) = x-\{(X-50)(x-y)/(X-Y)\}$$

The $IC_{50}$ values, the concentration of the compounds at percent inhibition 50%, are shown in the following Table 1. Compound No. in the Table 1 is the same as compound No. of the above example.

Table 1

| Compound No. | $IC_{50}$ (μg/mL) |
|---|---|
| I-3 | 0.40 |
| I-11 | 0.42 |
| I-12 | 0.43 |
| I-16 | 0.53 |
| I-17 | 1.6 |
| I-19 | 0.63 |
| I-20 | 0.32 |
| I-21 | 0.54 |
| I-22 | 0.59 |
| I-25 | 0.48 |
| I-29 | 1.0 |
| I-46 | 0.44 |
| I-47 | 0.35 |
| I-50 | 0.40 |

Table 1 (continued)

| Compound No. | $IC_{50}$ (μg/mL) |
|---|---|
| I-51 | 0.48 |

The inhibitory effects of the compounds and anti-retrovirus active substances of the present invention for HIV-1, HIV-2, SIVmac, and SIVagm integrases have been determined by the assay described below.
(1) Molt-4 cells (2 x $10^6$ cells) were infected with HIV-1 NL432 (4 x $10^6$ cpm), HIV-2 Rod (8 x $10^6$ cpm), SIVmac MA239 (8 x $10^6$ cpm) and SIVagm SA212 (8 x $10^6$ cpm), and incubated for 1 hr at room temperature.

Preparation of the viruses

① Preparation of HIV-1 virus : SW480 cells were cultured in DMEM medium supplemented with 10% fetal bovine serum using 25 $cm^2$ flask. HIV-1 infectious molecular DNA (40 μg), pNL432, transfected to the cells by calcium phosphate coprecipitation method, and the supernatant (2 mL) at 2-3 days post-transfection was infected to M8166 cells (1 x $10^6$ cells). The cells were cultured in 10 mL RPMI medium supplemented with 10% fetal bovine serum in $CO_2$ incubator at 37 °C. After apparent giant cells were observed, the cells were removed by centrifugation and then the supernatant was filtrated by 0.45 μm filter, measured RT activity, and stored at - 80 °C as HIV-1 stock virus.
② Preparation of HIV-2 virus: HIV-2 Rod was infected to M8166 cells (1 x $10^6$ cells). The cells were cultured in 10 mL RPMI medium supplemented with 10% fetal bovine serum in $CO_2$ incubator at 37 °C. After apparent giant cells were observed, the cells were removed by centrifugation and then the supernatant was filtrated by 0.45 μm filter, measured RT activity, and stored at - 80 °C as HIV-2 stock virus.
③ Preparation of SIVmac virus: SW480 cells were cultured in DMEM medium supplemented with 10% fetal bovine serum using 25cm$^2$ flask. SIVmac MA239 infectious molecular DNA (40 μg), pMA239, transfected to the cells by calcium phosphate coprecipitation method, and the supernatant (2 mL) at 2-3 days post-transfection was infected to CEMX174 cells (1 x $10^6$ cells). The cells were cultured in 10 mL RPMI medium supplemented with 10% fetal bovine serum in $CO_2$ incubator at 37 °C. After apparent giant cells were observed, the cells were removed by centrifugation and then the supernatant was filtrated by 0.45 μm filter, measured RT activity, and stored at - 80 °C as SIVmac stock virus.
④ Preparation of SIVagm virus: SW480 cells were cultured in DMEM medium supplemented with 10% fetal bovine serum using 25 $cm^2$ flask. SIVagm SA212 infectious molecular DNA (40 μg), pSA212, transfected to the cells by calcium phosphate coprecipitation methods, and the supernatant (2 mL) at 2-3 days post-transfection was infected to M8166 cells (1 x $10^6$ cells). The cells were cultured in 10 mL RPMI medium supplemented with 10% fetal bovine serum in $CO_2$ incubator at 37 °C. Every 3 days, the cells were separated from the supernatant by centrifugation and continued culture with new culture medium. The each supernatant was filtrated by 0.45 μm filter, measured RT activity, and stored at - 80 °C as SIVagm stock virus.

(2) The cells were washed twice to remove the viruses and suspended in 10 mL RPMI medium supplemented with 10% fetal bovine serum. The infected cells (100 μL) were plated in 96-well microtiter plates containing with the serial 5 fold dilution of compounds (100 μL) and cultured in $CO_2$ incubator at 37 °C.
(3) The supernatant at 5 days postinfection was harvested and measured the amount of the virus by RT activity.

Preparation of the solution for RT assay:

A reaction mixture (90 μL) were contained 50 mM Tris-HCl, pH 8.3, 150 mM KCl, 10 mM $MgCl_2$, 0.1% Nonidet P-40, 10 mM DTT (dithiothreitol), 5 μg/mL poly(rA), 5 μg/mL $(dT)_{12-18}$ and 1 μCi [$^3$H] dTTP at final concentration after mixture of a sample (10 μL).
The procedure of RT assay:

1. 10 μL of sample was plated to 3 wells of 96-well microtiter plate (triplicate assay).
2. Reaction mixture (90 μL) chilled at 4 °C was added to each well, mixed and then incubate at 37 °C for 3 hrs.
3. After incubation, the plate was chilled on ice, and passed through a DEAE-Filtermat using cell harvester. The filter was washed with 4.5% $Na_2HPO_4$ (20 sec) and $H_2O$ (10 sec).
4. The filter was dried at 95 °C for 15 min.
5. 10 mL of scintilator was added and sealed.

6. Radioactivity was measured by LKB Beta Plate scintillation spectroscopy.
7. RT activity (cpm/mL) was calculated from average of three wells.

(4) RT activity in the absence of medicine was calculated as 100% viral replication and then 50% inhibitory concentration ($EC_{50}$) of the sample was determined from the RT activity of each well as shown in Table 2.

Table 2

| Result $EC_{50}$ Unit ng/mL | | | | |
|---|---|---|---|---|
| | HIV-1 NL432 | HIV-2 ROD | SIVmac | SIVagm |
| I-16 | 57 | 35 | 57 | 44 |
| Zidovudine | 0.51 | 0.28 | 0.35 | 0.36 |
| Stavudine | 6.1 | 4.1 | 3.4 | 4.5 |
| Lamivudine | 8.4 | 22 | 9.1 | 22 |

As shown in table 2, the compound I-16, zidovudine, stavudine, and lamivudine showed antiviral activity against HIV-1, HIV-2, SIVmac and SIVagm. Thus, the anti-retrovirus composition which contains the compound represented by the formula (I) are effective medicine for not only HIV-1 but also HIV-2 and SIV infection.
The inhibitory effects of the compounds and anti-retrovirus active substances of the present invention for FIV integrases have been determined by the assay described below.
(1) The serial 5 fold diluted samples were plated in 24 well microtiter plate. Feline T-cell lines (MYA-1 cells: 4x $10^5$ cells/well) and feline immunodeficiency virus (FIV TM-2 strain: 3000 cpm/well) were added to each well. The cells were cultured with 1.5 mL RPMI medium supplemented with 10% fetal bovine serum, 2 µg/mL polybrene, 100 unit/mL human recombinant IL-2 and 50 µM 2-mercaptoethanol in $CO_2$ incubator at 37 °C.

① Preparation of FIV TM-2 virus: FIV TM-2 was infected to MYA-1 cells (1 x $10^6$ cells). The cells were cultured in 10 mL RPMI medium supplemented with 10% fetal bovine serum, 2 µg/mL polybrene, 100 unit/ml human recombinant IL-2 and 50 µM 2-mercaptoethanol in $CO_2$ incubator at 37 °C. Every 1-2 days, the cells were separated from the supernatant by centrifugation and continued culture with new culture medium. Each of the supernatant was filtrated by 0.45 µm filter, measured RT activity, and stored at as - 80 °C as FIV stock virus.
(2) Every 1-2 days, the supernatants were measured the amount of the virus by RT activity (as same as experimental example 2).
(3) Using from the data at 10 days postinfection, RT activity in the absence of medicine was calculated as 100% viral replication and then 50% inhibitory concentration ($EC_{50}$) of the sample was determined from the RT activity of each well as shown in Table 3.

Table 3

| Result $EC_{50}$ Unit ng/mL | |
|---|---|
| | $EC_{50}$ |
| I-16 | 300 |
| Zidovudine | 48 |

As shown in table 3, the compound I-16 showed antiviral activity against not only HIV-1 and SIV but also FIV. Thus, the anti-retrovirus composition which contains the compound represented by the formula (I) are effective medicine for FIV infection.

Reference example 2

The inhibitory effects of each anti-human immunodeficiency virus active substance against human immunodeficiency virus have been determined by MTT assay described below.

(1) Human T cell strain Molt-4 clone 8 persistently infected HIV (HTLV-IIIB strain) was cultivated in RPMI-1640 medium supplemented with 10% fatal bovine serum, and supernatant was filtered, measured RT activity, and stored at -80 °C. On the other hand, each human anti-immunodeficiency virus active substance was diluted to fixed concentration in culture medium, they were plated to 96-well microtiter plate. Subsequently, 100 µL (3.5 x

$10^4$ cells) of MT-4 cell suspension was plated to 96-well microtiter plate, and then above mentioned supernatant containing HIV, which was diluted by culture medium, was added 50 μL (60 pfu (plaque forming unit)) to 96-well microtiter plate.

(2) After cultivation at 37 °C for 5 days in $CO_2$ incubator, into all well was added 30 μL per well 3-(4,5-dimethylthiazol-3-yl)-2,5-diphenyltetrazolium bromide (MTT) 5 mg/mL) and PBS, and then incubated for 1 h.

In this time, alive cells reduced MTT and formazan was precipitated, therefore, 150 μL of cell supernatants were removed from all wells, and then 150 μL of lysate (10% triton X-100 and 0.4% (v/v) HCl-containing isopropanol) was added, and formazan was eluated on a plate shaker. Formazan was measured at OD 560 nm and 690 nm (reference wave length) by Microplate reader, the results were compared with reference. $EC_{50}$ was concentration of compound which inhibited 50% of cell damage caused by virus. The results were shown in Table 4.

Table 4

| Inhibitory activity against human virus Anti-human virus active substance | $EC_{50}$ (ng/mL) |
|---|---|
| Zidovudine | 1.8 |
| Didanosine | 640 |
| Zalcitabine | 17 |
| Lamivudine | 38 |
| Stavudine | 50 |
| Abacavir | 630 |
| Capravirine | 1.4 |
| Nevirapine | 20 |
| Loviride | 11 |
| Delavirdine | 12 |
| Emivirine | 4.5 |
| Efavirenz | 0.58 |
| Saquinavir | 3.7 |
| Indinavir | 14 |
| Ritonavir | 26 |
| Nelfinavir | 10 |

Experimental example

Tested samples (medicine 1 and medicine 2) were diluted with culture medium (10% FCS containing RPMI 1640 medium) to prepare appropriate concentration stocks of tested samples. The diluted of medicine 1 (50 μL) was plated into 96-well microtiter plate. And then the diluted medicine 2 (50 μL) was pipetted into them, total volume became 100 μL. In cell control (CC) wells 100 μL of the culture medium was added, and in virus control (VC) wells 50 μL of the culture medium was added. Three plates of same dilutions were prepared in order to measure synergistic effect (triple experiments).

The MT-4 cells (50 μL) diluted to suitable concentration (4 x $10^5$/mL) by above mentioned culture medium was added to each 96-well microtiter plate containing above mentiond tested sample, and the plate was mixed by plate shaker, and then was incubated in $CO_2$ incubator for 1 h.

The HIV-1 (50 μL) diluted to suitable concentration (moi=0.01) by above mentioned culture medium was added to each 96-well microtiter plate containing above mentioned tested sample and MT-4 cells, and the plate was mixed by plate shaker, and then was cultured in $CO_2$ incubator for 5 days.

After 5 days 30 μl of MTT solution (MTT was dissolved with PBS to be 5 mg/mL, filtered by 0.22 μm filter, and sterilized) was added to each 96-well microtiter plate, and then was incubated in $CO_2$ incubator for 1 h. 150 μL of supernatant was removed from each well without taking off the cells. 150 μL of lysate (500mL of 2-propanol, 50mL of Triton X and 2 mL of HCl were mixed properly) was added to 96-well microtiter plate, and then mixed properly by plate shaker until all cells were dissolved. The absorbance of the mixed to 96-well microtiter plate was measured at two wave length of 560 nm and 690 nm by microtitrer plate reader. By using MacSynergy II soft Synergy Volume ($\mu M^2$%, 99.9% confidence) of two kind of medicines, synergistic effects were determined. The methods of determination of synergistic effect by using Synergy Volume were described in ① Antiviral Research, 14, (1990), 181-206, ② Antimicrob. Agents, Chemother, (1997), 2165-2172, and the like.

Especially, it is preferred that synergistic effect is over 100 μM$^2$%.

Table 5

| | Synergy volumes (99.9% confidence) | | | |
|---|---|---|---|---|
| Combination (Medicine 1/Medicine 2) | Example 1 | Example 2 | Example 3 | Example 4 |
| Compound I-16/Didanosine | 234 | 214 | 159 | 254 |
| Compound I-16/Lamivudin | 200 | 193 | 365 | 181 |
| Compound I-16/Nevirapine | 68 | 245 | 257 | 243 |
| Compound I-16/Capravirine | 138 | 221 | 166 | 142 |
| Compound I-16/Nelfinavir | 129 | 283 | 136 | 243 |
| Compound I-16/Compound I-16 | ND | ND | 43 | 62 |
| ND: Not done. | | | | |

Furthermore, for example, a graph of the synergistic effect between a compound I-16 and zidovudine is shown in Figure 1.

**Industrial Applicability**

The combination of propenone derivatives and anti-retrovirus active substances can be efficiently inhibited increase of retrovirus replication by each medicine acting synergistically against retrovirus. A medicinal composition containing propenone derivatives and anti-retrovirus active substances of the present invention is a excellent anti-retrovirus composition.

**Claims**

1. An anti-retrovirus composition which contains as active ingredients a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances.

2. An anti-retrovirus composition as described in claim 1 wherein the anti-retrovirus active substance exhibits an synergistic effect in combination with a compound represented by the formula (I), a tautomer, a prodrug, a pharmaceutically acceptable salt, or a solvate thereof.

3. An anti-retrovirus composition as described in claim 1 or 2 wherein the anti-retrovirus active substance is one excluding integrase inhibitor.

4. An anti-retrovirus composition as described in any one of claims 1 to 3 wherein the anti-retrovirus active substance is a nucleoside type reverse transcriptase inhibitor, an non-nucleoside type reverse transcriptase inhibitor and/or a protease inhibitor.

5. An anti-retrovirus composition as described in any one of claims 1 to 4 wherein the anti-retrovirus active substance is zidovudine, didanosine, zalcitabine, stavudine, lamivudine, abacavir, tenofovir, tenofovir disoproxil, nevirapine, delavirdine, emivirine, loviride, efavirenz, trovirdine, capravirine, TIBO, talviraline, UC781, saquinavir, nelfinavir, ritonavir, indinavir, KNI-272, lopinavir, VX-478, VB-19026, BILA-2011-BS, A-77003, A-80987, DMP-323, and/or XM-450.

6. An anti-retrovirus composition as described in any one of claims 1 to 5 wherein the anti-retrovirus active substance is zidovudine, lamivudine, stavudine, nevirapine, capravirine, and/or nelfinavir.

7. An anti-retrovirus composition as described in any one of claims 1 to 6 wherein A of the compound represented by the formula (I) is optionally substituted furyl, optionally substituted thienyl, or optionally substituted pyridyl; and B of the compound represented by the formula (I) is optionally substituted triazolyl, optionally substituted tetrazolyl,

optionally substituted pyridyl, or optionally substituted oxazolyl.

**8.** An anti-retrovirus composition as described in any one of claims 1 to 7 wherein A of the compound represented by the formula (I) is 5-(4-fluorobenzyl)furan-2-yl; and B of the compound represented by the formula (I) is 1*H*-1,2,4-triazol-3-yl.

**9.** An anti-retrovirus composition as described in any one of claims 1 to 8 wherein retrovirus is human immunodeficiency virus.

**10.** A pharmaceutical composition which contains a compound, which exhibits an activity promoting anti-retrovirus activity of anti-retrovirus active substance, and is represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof.

**11.** A method for treating or preventing retrovirus infection, which comprises a simultaneous or continuous administration of the compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substances.

**12.** Use of a compound represented by the formula (I): A-C(=O)-CH=C(OH)-B wherein A is optionally substituted heteroaryl; B is optionally substituted heteroaryl or optionally substituted aryl; provided that cases wherein A and/or B are optionally substituted indol-3-yl are excluded; a tautomer, a prodrug, a pharmaceutically acceptable salt, or a solvate thereof, and one or more different type of anti-retrovirus active substance for preparation of a medicament for treating and preventing retrovirus infection.

Figure 1

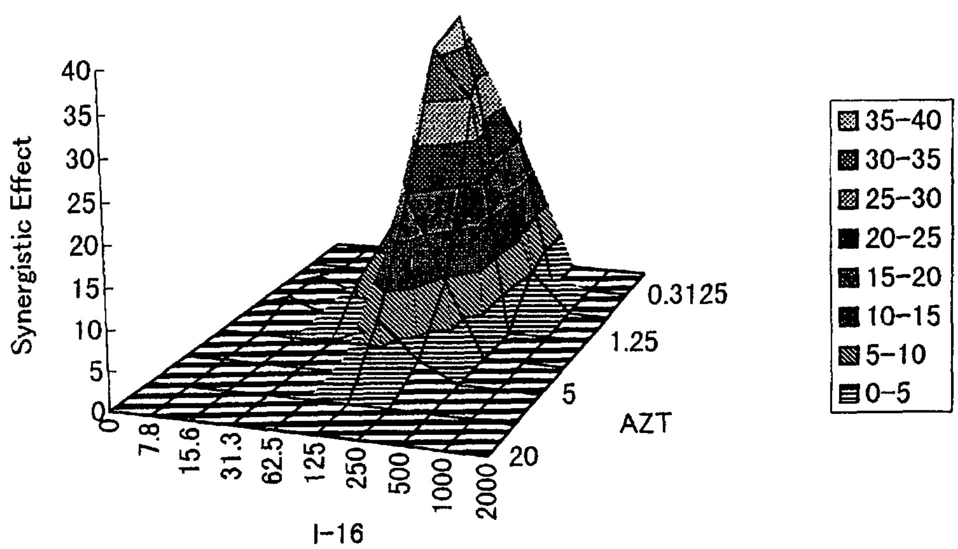
40
35
30
25
20
15
10
5
0
Synergistic Effect
0
7.8
15.6
31.3
62.5
125
250
500
1000
2000
I-16
0.3125
1.25
5
20
AZT
35-40
30-35
25-30
20-25
15-20
10-15
5-10
0-5

# INTERNATIONAL SEARCH REPORT

International application No.
PCT/JP01/04887

**A. CLASSIFICATION OF SUBJECT MATTER**
Int.Cl⁷ C07D403/06, C07D405/06, C07D409/06, C07D413/06, A61K31/4155, 41, 443, 506, 4439, 7072, 522, 675, A61K31/551, 496, 513, 165, 4196, 5517, 498, 341, 4725, 426, 496, 4709, 366, 519, 538, 4402, 472, A61K31/499, A61K45/00, A61P43/121, A61P31/18

According to International Patent Classification (IPC) or to both national classification and IPC

**B. FIELDS SEARCHED**

Minimum documentation searched (classification system followed by classification symbols)
Int.Cl⁷ C07D403/06, C07D405/06, C07D409/06, C07D413/06, A61K31/4155, 41, 443, 506, 4439, 7072, 522, 675, A61K31/551, 496, 513, 165, 4196, 5517, 498, 341, 4725, 426, 496, 4709, 366, 519, 538, 4402, 472, A61K31/499, A61K45/00, A61P43/121, A61P31/18

Documentation searched other than minimum documentation to the extent that such documents are included in the fields searched

Electronic data base consulted during the international search (name of data base and, where practicable, search terms used)
CA (STN), CAOLD (STN), REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 98/45268 A1 (Phizer Products Inc.), 15 October, 1998 (15.10.98), Full text & JP 2000-510481 A & AU 9862273 A & ZA 9802853 A & NO 9904791 A & EP 971894 A1 & CZ 9903489 A & CN 1254335 A & BR 9810733 A & SK 9901328 A & HU 200001243 A & MX 9909099 A | 1-10,12 |
| Y | WO 99/50245 A1 (Shionogi & Co., Ltd.), 07 October, 1999 (07.10.99), Full text & AU 9929581 A & BR 9909146 A & CZ 200003455 A & NO 200004787 A & EP 1069111 A1 & ZA 200004047 A | 1-10,12 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 July, 2001 (09.07.01) | 24 July, 2001 (24.07.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/04887

C (Continuation). DOCUMENTS CONSIDERED TO BE RELEVANT

| Category* | Citation of document, with indication, where appropriate, of the relevant passages | Relevant to claim No. |
|---|---|---|
| Y | EP 906756 A1 (Shionogi & Co., Ltd.), 07 April, 1999 (07.04.99), Full text & JP 9-536036 A Full text & WO 97/37657 A1 & AU 9719407 A & NO 9804616 A & CN 1220604 A & BR 9708592 A & HU 9902175 A & US 6083958 A & KR 2000005115 A | 1-10,12 |
| Y | EP 878194 A1 (Sankyo Company, Limited), 18 November, 1998 (18.11.98), Full text & JP 9-323932 A Full text & WO 97/27856 A1 & AU 9715564 A & NO 9803512 A & CZ 1214632 A & HU 9901642 A & MX 9806158 A & KR 99082201 A | 1-10,12 |
| PX | WO 01/11578 A1 (Merck & Co., Inc.), 04 January, 2001 (04.01.01), especially, Claims & AU 200058806 A | 1-10,12 |
| PX | WO 00/39086 A1 (Shionogi & Co., Ltd.), 06 July, 2000 (06.07.00), especially, Claims & AU 200017979 A | 1-10,12 |
| PA | WO 00/75122 A1 (Shionogi & Co., Ltd.), 14 December, 2000 (14.12.00), Full text & AU 200047827 A | 1-10,12 |

Form PCT/ISA/210 (continuation of second sheet) (July 1992)

**INTERNATIONAL SEARCH REPORT**

International application No.
PCT/JP01/04887

**Box I Observations where certain claims were found unsearchable (Continuation of item 1 of first sheet)**

This international search report has not been established in respect of certain claims under Article 17(2)(a) for the following reasons:

1. ☒ Claims Nos.: 11
because they relate to subject matter not required to be searched by this Authority, namely:
Claim 11 pertains to methods for treatment of the human body by therapy and thus relates to a subject matter which this International Searching Authority is not required to search.

2. ☒ Claims Nos.: 2
because they relate to parts of the international application that do not comply with the prescribed requirements to such an extent that no meaningful international search can be carried out, specifically:
(See extra sheet.)

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP01/04887

Continuation of Box No. I-2 of continuation of first sheet (1)

In claim 2, use is made of a substance "exerting a synergistic effect in combined use with the compound represented by the formula (I)". Considering the common technical knowledge at the application time, it is unclear what compounds, other than those disclosed in the description, can exert a synergistic effect in combined use with the compound represented by the formula (I). Thus, the particular scope thereof cannot be specified, which makes it impossible to practice any meaningful international search.

Form PCT/ISA/210 (extra sheet) (July 1992)